# EUROPEAN PATENT APPLICATION

(11) **EP 4 831 297 A1**
(43) Date of publication of application: **28.10.2026**
(21) Application number: 24907214.1
(22) Date of filing: 09.12.2024
(51) Int. Cl.: C12Q 1/6827, C12N 15/09, C12N 15/12, C12Q 1/6837, C12Q 1/6886

(54) **GENETIC MUTATION DETECTION METHOD**

(30) Priority: 22.12.2023 JP 2023216580
(71) Applicant: Toyo Kohan Co., Ltd., Tokyo 141-8260 (JP)
(72) Inventor: MURAMATSU, Marie, Tokyo 141-8260 (JP); MORIHIRO, Junichi, Tokyo 141-8260 (JP)
(74) Representative: Gleiss Große Schrell und Partner mbB
(86) International application number: PCT/JP2024/043341
(87) International publication number: WO 2025/134829

(57) **Abstract**

Gene mutations comprising a missense mutation and/or a nonsense mutation, and a silent mutation are analyzed with high accuracy. Signals are detected from a first probe corresponding to the missense mutation or the nonsense mutation in the gene mutations, a second probe corresponding to the silent mutation in the gene mutations, and a wild-type probe corresponding to a wild type, and the signal from the second probe is determined as a wild type for the gene mutations.

## Description

### Technical Field

The present invention relates to a method for detecting gene mutations comprising a missense mutation and/or a nonsense mutation, and a silent mutation.

### Background Art

A gene mutation broadly means a state in which a gene has developed a certain congenital or acquired abnormality. Examples of the gene mutation include a state in which a base constituting DNA is substituted, deleted, or added. Here, a gene is composed of a coding region that encodes a protein, and a non-coding region such as an expression regulatory region. A gene mutation is present in either the coding region or the non-coding region. In particular, a gene mutation present in the coding region comprises a missense mutation, in which the encoded amino acid changes, a nonsense mutation, in which a codon encoding a particular amino acid results in a stop codon, and a silent mutation, in which the encoded amino acid does not change.

A gene mutation can be a cause of a hereditary disease or a cancer, and is also related to the efficacy of a particular drug. In addition, a gene mutation is also related to a constitution such as obesity. Therefore, identifying a specific gene mutation (also referred to as genotype determination or genotyping) is an indispensable technique for diagnosing a hereditary disease and for knowing the efficacy of a drug.

Examples of a method for genotyping include, in addition to DNA sequencing, the SSCP (Single Strand Conformation Polymorphism) method, the RFLP (Restriction Fragment Length Polymorphism) method, the PCR (Polymerase Chain Reaction) method, the AFLP (Amplified Fragment Length Polymorphism) method, the ASO (Allele Specific Oligonucleotide) probe method, and a method in which binding to a DNA microarray or a DNA bead is detected. Among these techniques, in a method using a DNA microarray, the DNA microarray usually comprises, for a gene mutation to be detected, a mutant-type probe and a wild-type probe immobilized on a support.

In a method using a DNA microarray, first, using a primer having a fluorescent label, a region having a gene mutation to be detected is amplified by a nucleic acid amplification reaction. Thereafter, hybridization reaction is carried out between a nucleic acid fragment having the fluorescent label, and a mutant-type probe and a wild-type probe. In cases where the amplified nucleic acid fragment comprises a mutant type, fluorescence is observed from the mutant-type probe. Therefore, by observing fluorescence from the mutant-type probe and the wild-type probe, genotyping of the gene mutation can be carried out.

On the other hand, Patent Literature 1 is an example of a technique for determining the risk of onset of a disease by genotyping. Patent Literature 1 discloses that the risk of onset of primary central nervous system malignant lymphoma is determined based on a gene mutation in GRB2 and/or MYD88. Patent Literature 2 also discloses that, for treatment of diffuse large B-cell lymphoma (DLBCL), whether or not an agent for inhibiting BTK such as ibrutinib is to be used is determined based on modification (by gene mutation) of the 198th amino acid or the 265th amino acid in MYD88 and modification (by gene mutation) of the 196th amino acid in CD79B.

### Citation List

### Patent Literature

Patent Literature 1: WO 2016/098873
Patent Literature 2: JP 2017-523188 A

### Summary of Invention

### Technical Problem

However, there has been no established method that enables highly accurate analysis of gene mutations comprising a missense mutation and/or a nonsense mutation, and a silent mutation. In view of this, an object of the present invention is to provide a novel method for detecting gene mutations comprising a missense mutation and/or a nonsense mutation, and a silent mutation.

### Solution to Problem

The method for detecting gene mutations according to the present invention, which has achieved the above object, includes the following.
(1) A method for detecting gene mutations comprising a missense mutation and/or a nonsense mutation, and a silent mutation, comprising:
amplifying a nucleic acid region comprising the gene mutations;
hybridizing a nucleic acid fragment comprising the gene mutations with a first probe corresponding to the missense mutation or the nonsense mutation in the gene mutations, a second probe corresponding to the silent mutation in the gene mutations, and a wild-type probe corresponding to a wild type; and
detecting signals from the first probe, the second probe, and the wild-type probe,
wherein the signal from the second probe is determined as a wild type for the gene mutations.

(2) The method of (1), wherein a value for determination is calculated using the following formula: value for determination = [signal value from the second probe] / [signal value from the wild-type probe + signal value from the second probe], and a wild type is determined for the gene mutations when the calculated value for determination is more than a predetermined cutoff value.
(3) The method of (1), wherein a value for determination is calculated using the following formula: value for determination = [signal value from the first probe] / [signal value from the wild-type probe + signal value from the first probe], and the presence of the missense mutation and/or the nonsense mutation is determined for the gene mutations when the calculated value for determination is more than a predetermined cutoff value.
(4) The method of (3), wherein value for determinations are calculated using the formula for each of a plurality of the missense mutation and/or the nonsense mutation in the gene mutations, and the presence of each of the plurality of the missense mutation and/or nonsense mutation is determined.
(5) The method of (4), wherein a wild type is determined for the gene mutations when all of the values for determination calculated for each of the plurality of the missense mutation and/or nonsense mutation are less than the cutoff value.
(6) The method of (1), wherein the gene mutations are a missense mutation and/or a nonsense mutation occurring in a codon encoding a particular amino acid, and the first probe corresponds to a missense mutation and/or a nonsense mutation having a base substitution at the same position as a base substitution that results in the silent mutation for the amino acid.
(7) The method of (1), wherein the gene mutations are a missense mutation and/or a nonsense mutation occurring in a codon encoding a particular amino acid, and the first probe corresponds to a missense mutation and/or a nonsense mutation having a base substitution at a codon in which a base substitution that results in a silent mutation for the amino acid occurs.
(8) The method of (7), comprising using a plurality of first probes for all missense mutations and/or nonsense mutations having a base substitution in a codon in which a base substitution resulting in a silent mutation for the above amino acid occurs.
(9) The method of (1), wherein the gene mutations comprise a missense mutation and/or a nonsense mutation for the tyrosine residue at position 196 and a silent mutation for the tyrosine residue in the CD79B gene.
(10) The method of (9), wherein the missense mutation in the CD79B gene is at least one mutation selected from the group consisting of 589T>C, 589T>A, 589T>G, 590A>C, 590A>G and 590A>T, the nonsense mutation in the CD79B gene is 591C>G or 591C>A, and the silent mutation in the CD79B gene is 591C>T.
(11) The method of (10), wherein the first probe corresponding to the missense mutation and the nonsense mutation in the CD79B gene, the second probe corresponding to the silent mutation in the CD79B gene, and the wild-type probe corresponding to a wild-type corresponding to these missense mutation, nonsense mutation, and silent mutation comprise the base sequences shown in Table 1.

**[Table 1]**

| Probe type | Probe base sequence | SEQ ID NO: |
|---|---|---|
| Wild-type probe | CTTACCTCGTAGGTGTGAT | 10 |
| First probe | CTTACCTCGTGGGTGTGA | 14 |
| First probe | CTTACCTCGTTGGTGTGA | 19 |
| First probe | CTTACCTCGTCGGTGTGA | 24 |
| First probe | TTACCTCGGAGGTGTGAT | 32 |
| First probe | CTCGCAGGTGTGATCTT | 38 |
| First probe | CTTACCTCGAAGGTGTGAT | 41 |
| First probe | CTTACCTCCTAGGTGTGATCT | 50 |
| First probe | TCTCCTTACCTCTTAGGTGTG | 57 |
| Second probe | TCTCCTTACCTCATAGGTGTG | 66 |

The present description encompasses the content disclosed in Japanese Patent Application No. 2023-216580.

### Advantageous Effects of Invention

In the method for detecting gene mutations according to the present invention, a signal from a probe corresponding to a silent mutation and a signal from a probe corresponding to a wild type are detected together as a wild type. Therefore, according to the method for detecting gene mutations according to the present invention, a missense mutation and/or a nonsense mutation can be detected with high sensitivity.

### Brief Description of Drawings

Fig. 1 shows the results of measuring a fluorescence intensity value for various probes designed in Examples when a 100% mutation model specimen was used.
Fig. 2 shows the results of measuring a fluorescence intensity value for various probes designed in Examples when a 5% mutation model specimen was used.
Fig. 3 shows a value for determination calculated from a fluorescence intensity value for various probes designed in Examples when a 5% mutation model specimen was used.
Fig. 4 shows, for a wild-type probe (a gene mutation of the MYD88 gene) designed in Examples, the results of measuring a fluorescence intensity value and the results of calculating a value for determination when a wild-type sample, a 5% mutation model specimen, or genomic DNA was used.
Fig. 5 shows the results of measuring a fluorescence intensity value for a wild-type probe (a gene mutation of the CD79B gene) designed in Examples when a wild-type sample, a 5% mutation model specimen, or genomic DNA was used.
Fig. 6 shows a value for determination calculated from a fluorescence intensity value for a wild-type probe (a gene mutation of the CD79B gene) designed in Examples when a wild-type sample, a 5% mutation model specimen, or genomic DNA was used.
Fig. 7 shows the results (value for determination) of detecting a gene mutation in genomic DNA using various probes selected in Examples.
Fig. 8 shows a combination of a primer set for amplifying a region comprising a gene mutation of the CD79B gene tested in Examples and a primer set for amplifying a region comprising a gene mutation of the MYD88 gene tested in Examples.
Fig. 9 shows a relationship between the amount of nucleic acid amplification and the annealing temperature for each combination of primer sets.
Fig. 10 shows the results of confirming, by electrophoresis, a nucleic acid fragment comprised in a reaction solution for the combinations [5], [6], and [7] of primer sets.
Fig. 11 shows the results of carrying out PCR with the combinations [5], [6], and [7] of primer sets and detecting an amplified fragment by the fluorescence intensity of a wild-type probe and a mutant-type probe.
Fig. 12 shows the results of measuring a fluorescence intensity while changing the mixing ratio of a labeled primer and an unlabeled primer for the combinations [6] and [7] of primer sets.
Fig. 13 shows the concentration ratio between a forward primer and a reverse primer comprised in a primer set tested in Examples.
Fig. 14 shows a relationship between: the concentration ratio between a forward primer and a reverse primer comprised in a primer set (MYD88 gene) tested in Examples; and a fluorescence intensity value.
Fig. 15 shows a relationship between: the concentration ratio between a forward primer and a reverse primer comprised in a primer set (CD79B gene) tested in Examples; and the fluorescence intensity value.
Fig. 16 shows the results of measuring the fluorescence intensity from a mutant-type probe when a blocking nucleic acid (MYD88 gene) designed in Examples was used.
Fig. 17 shows the results of measuring the fluorescence intensity from a wild-type probe and a mutant-type probe when v2-2 or v2-3 among blocking nucleic acids (MYD88 gene) designed in Examples was used.
Fig. 18 shows a value for determination calculated from the fluorescence intensity measured with a wild-type probe and a mutant-type probe when v2-2 or v2-3 among blocking nucleic acids (MYD88 gene) designed in Examples was used.
Fig. 19 shows a value for determination calculated from the fluorescence intensity measured with a mutant-type probe when a blocking nucleic acid (CD79B gene) designed in Examples was used.
Fig. 20 shows a value for determination for each mutant-type probe calculated using v4-1 and v4-2 among blocking nucleic acids (CD79B gene) designed in Examples and using a wild-type model specimen or a 5% mutation model specimen.
Fig. 21 shows a fluorescence intensity value for each mutant-type probe calculated using v4-1 and v4-2 among blocking nucleic acids (CD79B gene) designed in Examples and using a wild-type model specimen or a 5% mutation model specimen.
Fig. 22 shows the results of calculating a value for determination for the blocking nucleic acid (CD79B gene) v4-2 designed in Examples while changing the concentration thereof.
Fig. 23 shows the results of measuring the fluorescence intensity from each mutant-type probe when the concentration of the blocking nucleic acid (CD79B gene) v4-2 designed in Examples was set to 500 nM, 750 nM, or 1000 nM and a 5% mutation model specimen was used.
Fig. 24 shows the results of calculating a value for determination for each mutant-type probe when the concentration of the blocking nucleic acid (CD79B gene) v4-2 designed in Examples was set to 500 nM or 750 nM and a wild-type model specimen or a 5% mutation model specimen was used.

### Description of Embodiments

The present invention is described below in detail.

In the method for detecting gene mutations according to the present invention, gene mutations comprising a silent mutation are detection targets. The term "gene mutations comprising a silent mutation" means gene mutations to bases constituting a codon encoding a particular amino acid, the gene mutations comprising both of the following: a mutant type that results in a silent mutation, which does not cause an amino acid change; and a mutant type that results in a missense mutation, which causes an amino acid change, and/or a mutant type that results in a nonsense mutation, which generates a stop codon.

Examples of such gene mutations include, but are not particularly limited to, gene mutations related to a disease, gene mutations related to drug efficacy, and gene mutations related to a constitution. However, the gene mutations related to a disease, drug efficacy, or constitution only needs to be possibly related to the disease, drug efficacy, or constitution, and is not necessarily required to have been scientifically proven to be related to the disease, drug efficacy, or constitution. For example, gene mutations related to a genetic disease or hereditary disease may be detection targets. The genetic disease or hereditary disease is intended to encompass diseases in general caused by mutation in a chromosome or gene. In some cases, such a genetic disease or hereditary disease occurs due to the presence of a particular congenital or acquired gene mutation. Therefore, detection of the gene mutations enables, or can contribute to, diagnosis of a genetic disease or hereditary disease.

Whether or not such gene mutations comprise a silent mutation can be determined by using a known database. For example, for gene mutations related to cancer, a catalog of somatic mutations in cancer (COSMIC: The Catalogue Of Somatic Mutations In Cancer) can be used to determine whether or not the gene mutations include a silent mutation, or gene mutations related to cancer having a silent mutation can be identified.

Examples of the gene mutations include mutations in the CD79B gene, which is related to diffuse large B-cell lymphoma (DLBCL), mutations in the BRAF gene, which is related to malignant melanoma and lung cancer, and mutations in the GNAS gene, which is related to intraductal papillary mucinous neoplasm.

Specifically, in the CD79B gene, gene mutations are known for the tyrosine at position 196 as counted from the N-terminus. The gene comprises the following three types of gene mutations as a result of base substitutions occurring in the codon (TAC) encoding the tyrosine: missense mutations that result in different amino acids, nonsense mutations that results in stop codons, and a silent mutation in which the encoded amino acid remains tyrosine.

Here, the missense mutations in the CD79B gene are a mutation in which the thymine at position 589 as counted from the 5'-end of the region encoding CD79B is substituted with cytosine (this mutation is denoted as 589T>C; the same applies hereinafter (A: adenine, G: guanine, C: cytosine, and T: thymine)), 589T>A, 589T>G, 590A>C, 590A>G, and 590A>T. Further, the nonsense mutations in the CD79B gene are 591C>G and 591C>A. Further, the silent mutation in the CD79B gene is 591C>T.

These mutations for the thymine at position 196 in the CD79B gene are summarized in Table 2. In the column "Type" of Table 2, W represents the wild-type sequence; M1 to M6 represent the missense mutations; M7 and M8 represent the nonsense mutations; and M9 represents the silent mutation.

**[Table 2]**

| Type | Amino acid | Base | | |
|---|---|---|---|---|
| | | 589 | 590 | 591 |
| W | Y196 | T | A | C |
| M1 | Y196H | C | A | C |
| M2 | Y196N | A | A | C |
| M3 | Y196D | G | A | C |
| M4 | Y196S | T | C | C |
| M5 | Y196C | T | G | C |
| M6 | Y196F | T | T | C |
| M7 | Y196*(STOP) | T | A | G |
| M8 | Y196*(STOP) | T | A | A |
| M9 | Y196= | T | A | T |

Further, specifically, in the BRAF gene, gene mutations are known for the valine at position 600 as counted from the N-terminus. The gene comprises the following two types of gene mutations as a result of base substitutions occurring in the codon (GTG) encoding the valine: missense mutations that result in different amino acids; and silent mutations in which the encoded amino acid remains valine.

Here, examples of the missense mutations in the BRAF gene include 1799T>A, 1799T>G, 1799T>C, 1798G>A, and 1798G>C. Examples of the silent mutations in the BRAF gene include 1800G>A and 1800G>T.

Further, specifically, in the GNAS gene, gene mutations are known for the arginine at position 844 as counted from the N-terminus. The gene comprises the following two types of gene mutations as a result of base substitutions occurring in the codon (CGT) encoding the arginine: missense mutations that result in different amino acids; and silent mutations in which the encoded amino acid remains arginine.

The gene mutations to which the method for detecting gene mutations according to the present invention is applicable are not limited to the gene mutations comprised in the CD79B gene, the gene mutations comprised in the BRAF gene, and the gene mutations comprised in the GNAS gene described above. The method is broadly applicable to gene mutations comprising a missense mutation and/or a nonsense mutation, and a silent mutation.

In a method for testing a gene according to the present invention, a missense mutation and/or a nonsense mutation for a particular amino acid is/are a detection target(s) for the gene mutations to be tested. In other words, among base mutations (for example, substitution mutations) occurring in a codon encoding a particular amino acid, a missense mutation and/or a nonsense mutation is/are a detection target(s) for the gene mutations to be tested. In particular, a method for testing a gene according to the present invention is characterized in that, when a silent mutation is detected among base mutations (for example, substitution mutations) occurring in a codon encoding a particular amino acid, the gene mutation is determined as a wild type.

For example, among missense mutations and/or nonsense mutations occurring in a codon encoding a particular amino acid, a missense mutation and/or a nonsense mutation having a base substitution at the same position as a base substitution that results in a silent mutation for the amino acid may be a detection target(s) for the gene mutations to be tested. Specifically, in the above-described gene mutations in the CD79B gene, the nonsense mutations 591C>G and 591C>A (M7 and M8 in Table 2), which are at the same position as the silent mutation 591C>T, may be detection targets.

Further, for example, among missense mutations and/or nonsense mutations occurring in a codon encoding a particular amino acid, a missense mutation and/or a nonsense mutation having a base substitution in a codon in which a base substitution that results in a silent mutation for the amino acid occurs may be a detection target(s) for the gene mutations to be tested. Specifically, for the above-described gene mutations in the CD79B gene, one or more mutations selected from the following mutations located in the codon comprising the silent mutation 591C>T may be detection targets: six missense mutations consisting of 589T>C, 589T>A, 589T>G, 590A>C, 590A>G, and 590A>T (M1 to M6 in Table 2) and two nonsense mutations consisting of 591C>G and 591C>A (M7 and M8 in Table 2). All of the missense mutations 589T>C, 589T>A, 589T>G, 590A>C, 590A>G, and 590A>T, and the nonsense mutations 591C>G and 591C>A (M1 to M8 in Table 2), may be set as the missense mutations and nonsense mutations to be detected.

In the method for detecting gene mutations according to the present invention, a silent mutation, and a missense mutation and/or a nonsense mutation in these gene mutations to be tested are detected using probes. Specifically, a nucleic acid region comprising the gene mutations to be tested is amplified, and the presence of a silent mutation, a missense mutation and/or a nonsense mutation is confirmed based on hybridization between the obtained nucleic acid fragment and probes. In cases where the absence of any of these silent mutation, missense mutation, and nonsense mutation is confirmed, the gene mutation comprised in the amplified fragment is identified as a wild type.

Here, a probe for detecting a missense mutation and/or a nonsense mutation in the gene mutations to be tested is referred to as a first probe, and a probe for detecting a silent mutation in the gene mutations is referred to as a second probe. A probe for detecting a wild type for the gene mutations is referred to as a wild-type probe. These first probe, second probe, and wild-type probe can be appropriately designed based on the base sequence(s) of the missense mutation and/or nonsense mutation in the gene mutations to be tested, the base sequence of the silent mutation in the gene mutations, and the base sequence of the wild type.

The base length of each of these probes is not particularly limited. The base length may be, for example, 10 to 30 bases, and is preferably 15 to 25 bases. Each probe is preferably nucleic acid, more preferably DNA. The DNA comprises both a double-stranded DNA and a single-stranded DNA, but is preferably a single-stranded DNA. The probe can be obtained, for example, by chemical synthesis using a nucleic acid synthesizer. As the nucleic acid synthesizer, an apparatus called a DNA synthesizer, a fully automatic nucleic acid synthesizer, a nucleic acid automatic synthesizer, or the like may be used.

The probe designed as described above is preferably used in the form of a microarray (for example, a DNA chip) by immobilizing the 5'-end thereof on a support. At this time, the microarray has the first probe, the second probe, and the wild-type probe for the gene mutations to be tested. The microarray may have the first probe, the second probe, and the wild-type probe for each of a plurality of types of gene mutations. The microarray can be produced by immobilizing the first probe, the second probe, and the wild-type probe described above on a support.

As a material of the support, materials known in the art can be used, and there is no particular limitation of the material. Examples of the material include conductor materials such as noble metals including platinum, platinum black, gold, palladium, rhodium, silver, mercury, tungsten, and compounds thereof, and carbons represented by graphite and carbon fibers; silicon materials represented by single-crystal silicon, amorphous silicon, silicon carbide, silicon oxide, silicon nitride, and the like, and composite materials of these silicon materials represented by SOI (silicon-on-insulator) and the like; inorganic materials such as glass, quartz glass, alumina, sapphire, ceramics, forsterite, and photosensitive glass; and organic materials such as polyethylene, ethylene, polypropylene, cyclic polyolefin, polyisobutylene, polyethylene terephthalate, unsaturated polyester, fluorine-containing resin, polyvinyl chloride, polyvinylidene chloride, polyvinyl acetate, polyvinyl alcohol, polyvinyl acetal, acrylic resin, polyacrylonitrile, polystyrene, acetal resin, polycarbonate, polyamide, phenol resin, urea resin, epoxy resin, melamine resin, styrene-acrylonitrile copolymer, acrylonitrile-butadiene-styrene copolymer, polyphenylene oxide, and polysulfone. The shape of the support is also not particularly limited, and is preferably a plate-like shape.

As the support, a support having a carbon layer and a chemical modification group on a surface is preferably used. The support having a carbon layer and a chemical modification group on a surface includes a support having a carbon layer and a chemical modification group on a surface of a substrate, and a support having a chemical modification group on a surface of a substrate consisting of a carbon layer. As a material of the substrate, materials known in the art can be used without particular limitation, and the same materials as those exemplified above as support materials can be used.

In the microarray, a support having a fine plate-like structure is preferably used. The shape is not limited, and may be a rectangular shape, a square shape, a circular shape, or the like. It has a side length of usually 1 to 75 mm, preferably 1 to 10 mm, more preferably 3 to 5 mm. Because a support having a fine plate-like structure can be easily produced, it is preferred to use a substrate consisting of a silicon material or a resin material, and a support having a carbon layer and a chemical modification group on a surface of a substrate consisting of single-crystal silicon is more preferred. The single-crystal silicon also includes one in which the orientation of the crystal axis is changed very slightly from part to part (which is sometimes referred to as a mosaic crystal), and one comprising disorder on an atomic scale (lattice defect).

The carbon layer to be formed on the substrate is not particularly limited. It is preferred to use any of a synthetic diamond, a high-pressure synthetic diamond, a natural diamond, a soft diamond (for example, diamond-like carbon), an amorphous carbon, and a carbon-based substance (for example, graphite, fullerene, or carbon nanotube), a mixture thereof, or a laminated product thereof. A carbide such as hafnium carbide, niobium carbide, silicon carbide, tantalum carbide, thorium carbide, titanium carbide, uranium carbide, tungsten carbide, zirconium carbide, molybdenum carbide, chromium carbide, or vanadium carbide may also be used. Here, the soft diamond is a general term for an incomplete diamond structure that is a mixture of diamond and carbon, such as the so-called diamond-like carbon (DLC: Diamond Like Carbon), and the mixing ratio thereof is not particularly limited. The carbon layer is advantageous from the viewpoint of the fact that it has excellent chemical stability and can withstand subsequent reactions for introduction of a chemical modification group and binding to an analyte substance, the fact that the carbon layer binds to an analyte substance through electrostatic bonding and hence the binding has flexibility, the fact that the carbon layer is transparent to UV in a detection system due to the absence of UV absorption, and the fact that energization is possible during electroblotting. Further, the carbon layer is advantageous from the viewpoint of the fact that non-specific adsorption is low in its binding reaction with an analyte substance. As described above, a support in which the substrate itself consists of a carbon layer may also be used.

The carbon layer can be formed by a known method. Examples of the method include the microwave plasma CVD (Chemical vapor deposit) method, the ECRCVD (Electric cyclotron resonance chemical vapor deposit) method, the ICP (Inductive coupled plasma) method, the direct-current sputtering method, the ECR (Electric cyclotron resonance) sputtering method, the ionized vapor deposition method, the arc vapor deposition method, the laser vapor deposition method, the EB (Electron beam) vapor deposition method, and the resistance-heating vapor deposition method.

In the high-frequency plasma CVD method, a raw material gas (methane) is decomposed by glow discharge generated between electrodes by high frequency, and a carbon layer is synthesized on a substrate. In the ionized vapor deposition method, a raw material gas (benzene) is decomposed and ionized using thermoelectrons generated by a tungsten filament, and a carbon layer is formed on a substrate by a bias voltage. The carbon layer may also be formed by the ionized vapor deposition method in a mixed gas consisting of 1 to 99 vol% hydrogen gas and the balance (99 to 1 vol%) methane gas.

In the arc vapor deposition method, a direct-current voltage is applied between a solid graphite material (cathode evaporation source) and a vacuum vessel (anode) to cause arc discharge in vacuum and thereby generate plasma of carbon atoms from the cathode, and a bias voltage that is more negative than the evaporation source is applied to the substrate to accelerate carbon ions in the plasma toward the substrate. By this, a carbon layer can be formed.

In the laser vapor deposition method, a carbon layer can be formed, for example, by irradiating a graphite target plate with Nd:YAG laser (pulse oscillation) light to melt the plate and depositing carbon atoms on a glass substrate.

When a carbon layer is formed on the surface of the substrate, the thickness of the carbon layer is usually from a monolayer to about 100 µm. In cases where the layer is too thin, the surface of the underlying substrate may be locally exposed, while in cases where the layer is too thick, productivity becomes poor. Therefore, the thickness is preferably 2 nm to 1 µm, more preferably 5 nm to 500 nm.

By introducing a chemical modification group onto the surface of the substrate on which the carbon layer has been formed, a probe can be firmly immobilized on the support. The chemical modification group to be introduced can be appropriately selected by those skilled in the art and is not particularly limited. Examples of the chemical modification group include an amino group, a carboxyl group, an epoxy group, a formyl group, a hydroxyl group, and an active ester group.

The amino group can be introduced, for example, by irradiating the carbon layer with ultraviolet light in ammonia gas or by performing plasma treatment of the carbon layer. Alternatively, the amino group can be introduced by chlorinating the carbon layer by irradiation with ultraviolet light in chlorine gas and then further performing irradiation with ultraviolet light in ammonia gas. Alternatively, the amino group can be introduced by reacting the chlorinated carbon layer with a polyvalent amine gas such as methylenediamine or ethylenediamine.

The carboxyl group can be introduced, for example, by reacting an appropriate compound with a carbon layer that has been aminated as described above. Examples of the compound used for introducing the carboxyl group include halocarboxylic acids represented by the formula: X-R1-COOH (wherein X represents a halogen atom, and R1 represents a divalent hydrocarbon group having 10 to 12 carbon atoms), such as chloroacetic acid, fluoroacetic acid, bromoacetic acid, iodoacetic acid, 2-chloropropionic acid, 3-chloropropionic acid, 3-chloroacrylic acid, and 4-chlorobenzoic acid; dicarboxylic acids represented by the formula: HOOC-R2-COOH (wherein R2 represents a single bond or a divalent hydrocarbon group having 1 to 12 carbon atoms), such as oxalic acid, malonic acid, succinic acid, maleic acid, fumaric acid, and phthalic acid; polyvalent carboxylic acids such as polyacrylic acid, polymethacrylic acid, trimellitic acid, and butanetetracarboxylic acid; keto acids or aldehyde acids represented by the formula: R3-CO-R4-COOH (wherein R3 represents a hydrogen atom or a divalent hydrocarbon group having 1 to 12 carbon atoms, and R4 represents a divalent hydrocarbon group having 1 to 12 carbon atoms); monohalides of dicarboxylic acids represented by the formula: X-OC-R5-COOH (wherein X represents a halogen atom, and R5 represents a single bond or a divalent hydrocarbon group having 1 to 12 carbon atoms), such as succinic acid monochloride and malonic acid monochloride; and acid anhydrides such as phthalic anhydride, succinic anhydride, oxalic anhydride, maleic anhydride, and butanetetracarboxylic anhydride.

The epoxy group can be introduced, for example, by reacting an appropriate polyvalent epoxy compound with a carbon layer that has been aminated as described above. Alternatively, the introduction can be achieved by reacting an organic peracid with a carbon-carbon double bond contained in a carbon layer. Examples of the organic peracid include peracetic acid, perbenzoic acid, diperoxyphthalic acid, performic acid, and trifluoroperacetic acid.

The formyl group can be introduced, for example, by reacting glutaraldehyde with a carbon layer that has been aminated as described above.

The hydroxyl group can be introduced, for example, by reacting water with a carbon layer that has been chlorinated as described above.

The active ester group means a group of ester that has an electron-withdrawing group having high acidity on the alcohol side of an ester group, and that activates a nucleophilic reaction, that is, an ester group having high reaction activity. The active ester group is an ester group that has an electron-withdrawing group on the alcohol side thereof and that has been activated more than an alkyl ester. The active ester group has reactivity with groups such as an amino group, a thiol group, and a hydroxyl group. More specifically, phenol esters, thiophenol esters, N-hydroxylamine esters, cyanomethyl esters, esters of heterocyclic hydroxy compounds, and the like are known as active ester groups having much higher activity than alkyl esters and the like. More specifically, examples of the active ester group include a p-nitrophenyl group, an N-hydroxysuccinimide group, a succinimide group, a phthalimide group, and a 5-norbornene-2,3-dicarboxyimide group. In particular, an N-hydroxysuccinimide group is preferably used.

The active ester group can be introduced, for example, by converting a carboxyl group that has been introduced as described above into an active ester using an agent for dehydration condensation such as cyanamide or carbodiimide (for example, 1-[3-(dimethylamino)propyl]-3-ethylcarbodiimide) and a compound such as N-hydroxysuccinimide. By this treatment, a group in which an active ester group such as an N-hydroxysuccinimide group is bonded to the end of the hydrocarbon group via an amide bond can be formed (JP 2001-139532 A).

A microarray having a probe immobilized on a support can be produced by dissolving the probe in a spotting buffer to prepare a spotting solution, dispensing the prepared spotting solution into a 96-well or 384-well plastic plate, and spotting the dispensed solution on the support using a spotter apparatus or the like. Alternatively, the spotting solution may be manually spotted using a micropipetter.

After the spotting, incubation is preferably carried out in order to allow the reaction of binding the probe to the support to proceed. The incubation is carried out at a temperature of usually -20 to 100°C, preferably 0 to 90°C, for usually 0.5 to 16 hours, preferably 1 to 2 hours. The incubation is desirably carried out under an atmosphere with high humidity, for example, under conditions with a humidity of 50 to 90%. The incubation is preferably followed by washing with a washing solution (for example, 50 mM TBS/0.05% Tween 20, 2×SSC/0.2% SDS solution, ultrapure water, or the like) in order to remove DNA not bound to the support.

By using a microarray composed as described above, for example, the presence of a missense mutation and/or a nonsense mutation or the presence of a wild type can be determined for gene mutations in a subject to be diagnosed.

Specifically, testing of the gene mutations comprises the steps of: extracting DNA from a sample derived from the subject to be diagnosed; amplifying a region comprising the gene mutations to be tested, using the extracted DNA as a template; hybridizing an amplified nucleic acid fragment with a first probe, a second probe, and a wild-type probe using the microarray described above; and detecting signals from the first probe, the second probe, and the wild-type probe.

The subject to be diagnosed is usually a human, and the race and the like thereof are not particularly limited. The subject is, in particular, an Asian subject, preferably an East Asian subject, especially preferably a Japanese subject. The subject to be diagnosed can also be a patient suspected of having a disease associated with the gene mutations to be tested.

The sample derived from the subject to be diagnosed is not particularly limited. Examples of the sample include blood-related samples (blood, serum, plasma, and the like), lymph fluid, feces, cancer cells, and a homogenate and an extract of a tissue or an organ.

First, DNA is extracted from a sample collected from the subject to be diagnosed. The extraction means is not particularly limited. For example, a DNA extraction method using phenol/chloroform, ethanol, sodium hydroxide, CTAB, or the like can be used.

Subsequently, an amplification reaction is carried out using the obtained DNA as a template, to amplify a region comprising the gene mutations to be tested. As the amplification reaction, polymerase chain reaction (PCR), LAMP (Loop-Mediated Isothermal Amplification), the ICAN (Isothermal and Chimeric primer-initiated Amplification of Nucleic acids) method, or the like can be applied. In the amplification reaction, a label is preferably added so as to enable identification of the region after the amplification. At this time, the method for labeling the amplified nucleic acid is not particularly limited. For example, a method in which a primer used in the amplification reaction is labeled in advance may be used, or a method in which a labeled nucleotide is used as a substrate in the amplification reaction may be used. The labeling substance is not particularly limited. For example, a radioactive isotope, a fluorescent dye, or an organic compound such as digoxigenin (DIG) or biotin can be used.

This reaction system comprises a buffer necessary for nucleic acid amplification and labeling, a thermostable DNA polymerase, a primer specific to the amplification region, a labeled nucleotide triphosphate (specifically, a nucleotide triphosphate to which a fluorescent label or the like has been added), a nucleotide triphosphate, magnesium chloride, and the like.

The nucleic acid fragment amplified by the primers is not particularly limited as long as it comprises a region corresponding to the designed probe, and is preferably a fragment of, for example, not more than 1 kbp, more preferably not more than 800 bp, still more preferably not more than 500 bp, especially preferably not more than 350 bp.

By carrying out hybridization reaction between the amplified nucleic acid obtained as described above and the probes immobilized on the support, and detecting occurrence of hybridization of the amplified nucleic acid with the first probe, the second probe, and the wild-type probe, the gene mutations in the subject to be diagnosed can be tested. Thus, occurrence of hybridization of the amplified nucleic acid with the first probe, the second probe, and the wild-type probe can be measured, for example, by detecting the label. In particular, in the present invention, when the second probe detects a silent mutation in the gene mutations, a wild type is determined for the gene mutations.

When, for example, a fluorescent label is used, a signal from the label can be quantified as a signal intensity by detecting the fluorescence signal using a fluorescence scanner and analyzing the detected fluorescence signal with image analysis software. The hybridization reaction is preferably carried out under stringent conditions. The term "stringent conditions" means conditions under which a specific hybrid is formed without formation of a non-specific hybrid, and means, for example, conditions in which hybridization reaction is carried out at 47 to 52°C (specifically, 51.5°C) for 15 to 90 minutes (specifically, 30 minutes) followed by 30 times of washing under conditions of 0.1×SSC/0.1% SDS at 25°C, and 80 times of washing under conditions of 1×SSC at 25°C. In cases where the chain length of the probe is short, the hybridization temperature is more preferably lower than this, while in cases where the chain length is long, the hybridization temperature is more preferably higher than this. Needless to say, as the salt concentration increases, the temperature for specific hybridization increases, while as the salt concentration decreases, the temperature for specific hybridization decreases.

In cases where a microarray comprising the first probe, the second probe, and the wild-type probe for the gene mutations described above is used, the gene mutations can be tested using signal intensities from the first probe, the second probe, and the wild-type probe. Specifically, the signal intensities from the first probe, the second probe, and the wild-type probe are each measured, and a value for determination for evaluating the signal intensity derived from the first probe or the second probe is calculated. Examples of the calculation of the value for determination include a method in which Formula 1: [signal intensity derived from the first probe] / ([signal intensity derived from the wild-type probe] + [signal intensity derived from the first probe]) is used to evaluate the missense mutation and/or nonsense mutation corresponding to the first probe, and Formula 2: [signal intensity derived from the second probe] / ([signal intensity derived from the wild-type probe] + [signal intensity derived from the second probe]) is used to evaluate the silent mutation corresponding to the second probe.

Thereafter, the value for determination calculated by Formula 1 above is compared with a predetermined threshold value (cutoff value), and in cases where the value for determination is higher than the threshold value, the amplified nucleic acid is determined to comprise the missense mutation and/or nonsense mutation corresponding to the first probe for the gene mutations, while in cases where the value for determination is lower than the threshold value, the amplified nucleic acid is determined not to comprise the missense mutation and/or nonsense mutation. Further, the value for determination calculated by Formula 2 above is compared with a predetermined threshold value (cutoff value), and in cases where the value for determination is higher than the threshold value, the amplified nucleic acid is determined to comprise the silent mutation corresponding to the second probe for the gene mutations, while in cases where the value for determination is lower than the threshold value, the amplified nucleic acid is determined not to comprise the silent mutation.

Then, in cases where the amplified nucleic acid is determined to comprise the silent mutation on the basis of Formula 2 above, a wild type is determined for the gene mutations to be tested. Further, in cases where the amplified nucleic acid is determined not to comprise the missense mutation and/or nonsense mutation on the basis of Formula 1 above, and in addition, not to comprise the silent mutation on the basis of Formula 2 above, a wild type is determined for the gene mutations to be tested.

Here, the threshold value is not particularly limited, and may be defined, for example, on the basis of a value for determination calculated by Formula 1 or Formula 2 above using a specimen in which a wild type has been confirmed for the gene mutations. More specifically, a plurality of values for determination may be calculated using a plurality of specimens in which a wild type has been confirmed for the gene mutations, and the mean value + 3σ (σ: standard deviation) may be used as the threshold value. The mean value + 2σ or the mean value + σ may also be used as the threshold value.

As described above, by using a microarray comprising a first probe, a second probe, and a wild-type probe for testing predetermined gene mutations, a mutant type (missense mutation and/or nonsense mutation) or a wild type can be identified for the gene mutations with high accuracy. For example, by detecting the mutant types M1 to M8 shown in Table 2 in the CD79B gene (M1 to M6 are missense mutations, and M7 and M8 are nonsense mutations), and determining a wild type in the case of M9, diffuse large B-cell lymphoma (DLBCL) with which the above gene mutations in the CD79B gene are associated can be diagnosed with high accuracy.

Table 3 summarizes a wild-type probe, first probes, and a second probe designed for detecting the wild type, the mutant types M1 to M8, and the silent mutation M9 shown in Table 2 in the CD79B gene. However, the probes are not particularly limited to these. In the base sequences of the probes described in Table 3, the portion corresponding to the codon encoding the 196th amino acid as counted from the N-terminus is underlined.

**[Table 3]**

| Wild type (W)/ Mutant type (M) | Probe base sequence | SEQ ID NO: |
|---|---|---|
| W | CTTACCTCGTAGGTGTGAT | 10 |
| M1 | CTTACCTCGTGGGTGTGA | 14 |
| M2 | CTTACCTCGTTGGTGTGA | 19 |
| M3 | CTTACCTCGTCGGTGTGA | 24 |
| M4 | TTACCTCGGAGGTGTGAT | 32 |
| M5 | CTCGCAGGTGTGATCTT | 38 |
| M6 | CTTACCTCGAAGGTGTGAT | 41 |
| M7 | CTTACCTCCTAGGTGTGATCT | 50 |
| M8 | TCTCCTTACCTCTTAGGTGTG | 57 |
| M9 | TCTCCTTACCTCATAGGTGTG | 66 |

When the wild-type probe, the first probes (M1 to M8), and the second probe (M8) shown in Table 2 are used, in cases where [signal intensity derived from the second probe] / ([signal intensity derived from the wild-type probe] + [signal intensity derived from the second probe]) is higher than a threshold value, the silent mutation can be determined to be present for the codon encoding the 196th amino acid as counted from the N-terminus, and hence the codon can be determined to be a wild type. As a result, the presence of a missense mutation and/or nonsense mutation in the gene mutations in the CD79B gene can be identified with high accuracy, and hence diffuse large B-cell lymphoma (DLBCL) can be diagnosed with high accuracy.

### Examples

The present invention is described below in more detail by way of Examples. However, the technical scope of the present invention is not limited to the following Examples.

### <Sequence Design>

In the present Example, for gene mutations in the MYD88 gene and gene mutations in the CD79B gene, primer sets for amplifying regions comprising the gene mutations, probes for detecting a wild type or a mutant type comprised in a nucleic acid fragment amplified with the primer sets, and blocking nucleic acids corresponding to wild types for the gene mutations in the MYD88 gene and the gene mutations in the CD79B gene were designed.

Table 4 shows the probes corresponding to the gene mutations in the MYD88 gene designed in the present Example. In the "Wild type (W)/Mutant type (M)" column of Table 4, W represents a probe corresponding to a wild type, and M represents a probe corresponding to a mutant type.

**[Table 4]**

| Wild type (W)/ Mutant type (M) | Name | Base sequence [5'→3'] | SEQ ID NO: |
|---|---|---|---|
| W | MYD88_W_v1 | CAGAAGCGACTGATCCCCATC | 1 |
| W | MYD88_W_v2-1 | AGCGACTGATCCCCAT | 2 |
| W | MYD88_W_v2-2 | AAGCGACTGATCCCCAT | 3 |
| W | MYD88_W_v2-3 | CAGAAGCGACTGATCCC | 4 |
| M | MYD88_M_v1 | CAGAAGCGACCGATCCCCATCA | 5 |
| M | MYD88_M_v2-1 | GAAGCGACCGATCCC | 6 |
| M | MYD88_M_v2-2 | CAGAAGCGACCGATCC | 7 |

Tables 5 and 6 show the probes corresponding to the gene mutations in the CD79B gene designed in the present Example. In the "Wild type (W)/Mutant type (M)" column of Tables 5 and 6, W represents a probe corresponding to a wild type, and M1 to M9 represent probes corresponding to mutant types. The notations M1 to M9 are in accordance with Table 3.

**[Table 5]**

| Wild type (W)/ Mutant type (M) | Name | Base sequence [5'→3'] | SEQ ID NO: |
|---|---|---|---|
| W | CD79B_W_v1 | TCCTTACCTCGTAGGTGTGATCTTC | 8 |
| W | CD79B_W_v2 | CTTACCTCGTAGGTGTGATC | 9 |
| W | CD79B_W_v3-1 | CTTACCTCGTAGGTGTGAT | 10 |
| W | CD79B_W_v3-2 | TTACCTCGTAGGTGTGATC | 11 |
| M1 | CD79B_M1_v1 | TCTCCTTACCTCGTGGGTGTGATCTTC | 12 |
| M1 | CD79B_M1_v2-1 | CTTACCTCGTGGGTGTG | 13 |
| M1 | CD79B_M1_v2-2 | CTTACCTCGTGGGTGTGA | 14 |
| M2 | CD79B_M2_v1 | TCTCCTTACCTCGTTGGTGTGATCTTC | 15 |
| M2 | CD79B_M2_v2-1 | CTTACCTCGTTGGTGTGATC | 16 |
| M2 | CD79B_M2_v2-2 | CCTTACCTCGTTGGTGTGATC | 17 |
| M2 | CD79B_M2_v2-3 | CCTTACCTCGTTGGTGTGAT | 18 |
| M2 | CD79B_M2_v3-1 | CTTACCTCGTTGGTGTGA | 19 |
| M2 | CD79B_M2_v3-2 | TCTCCTTACCTCGTTGGTGT | 20 |
| M2 | CD79B_M2_v3-3 | CCTCGTTGGTGTGATCTT | 21 |
| M3 | CD79B_M3_v1 | TCTCCTTACCTCGTCGGTGTGATCTTC | 22 |
| M3 | CD79B_M3_v2-1 | CTTACCTCGTCGGTGTG | 23 |
| M3 | CD79B_M3_v2-2 | CTTACCTCGTCGGTGTGA | 24 |
| M4 | CD79B_M4_v1 | TCTCCTTACCTCGGAGGTGTGATCTTC | 25 |
| M4 | CD79B_M4_v2-1 | CTTACCTCGGAGGTGTG | 26 |
| M4 | CD79B_M4_v2-2 | CTTACCTCGGAGGTGTGA | 27 |
| M4 | CD79B_M4_v3-1 | CTTACCTCGGAGGTGT | 28 |
| M4 | CD79B_M4_v3-2 | CCTCGGAGGTGTGATC | 29 |
| M4 | CD79B_M4_v4-1 | TTACCTCGGAGGTGTG | 30 |
| M4 | CD79B_M4_v4-2 | TTACCTCGGAGGTGT | 31 |
| M4 | CD79B_M4_v4-3 | TTACCTCGGAGGTGTGAT | 32 |
| M4 | CD79B_M4_v4-4 | CTCGGAGGTGTGATCTT | 33 |
| M5 | CD79B_M5_v1 | TCTCCTTACCTCGCAGGTGTGATCTTC | 34 |
| M5 | CD79B_M5_v2 | CTTACCTCGCAGGTGTG | 35 |
| M5 | CD79B_M5_v5-1 | CCTCGCAGGTGTGATC | 36 |
| M5 | CD79B_M5_v5-2 | TTACCTCGCAGGTGTGAT | 37 |
| M5 | CD79B_M5_v5-3 | CTCGCAGGTGTGATCTT | 38 |

**[Table 6]**

| Wild type (W)/ Mutant type (M) | Name | Base sequence [5'→3'] | SEQ ID NO: |
|---|---|---|---|
| M6 | CD79B_M6_v1 | TCTCCTTACCTCGAAGGTGTGATCTTC | 39 |
| M6 | CD79B_M6_v2-1 | CTTACCTCGAAGGTGTGATC | 40 |
| M6 | CD79B_M6_v2-2 | CTTACCTCGAAGGTGTGAT | 41 |
| M6 | CD79B_M6_v2-3 | CCTTACCTCGAAGGTGTGAT | 42 |
| M6 | CD79B_M6_v3-1 | CTTACCTCGAAGGTGTG | 43 |
| M6 | CD79B_M6_v3-2 | CCTCGAAGGTGTGATC | 44 |
| M6 | CD79B_M6_v4-1 | CCTCGAAGGTGTGAT | 45 |
| M6 | CD79B_M6_v4-2 | TTACCTCGAAGGTGTGAT | 46 |
| M6 | CD79B_M6_v4-3 | CTCGAAGGTGTGATCTTC | 47 |
| M6 | CD79B_M6_v4-4 | CTTACCTCGAAGGTGTGA | 48 |
| M7 | CD79B_M7_v1 | CTCTCCTTACCTCCTAGGTGTGATCTTCC | 49 |
| M7 | CD79B_M7_v2-1 | CTTACCTCCTAGGTGTGATCT | 50 |
| M7 | CD79B_M7_v2-2 | CCTTACCTCCTAGGTGTGA | 51 |
| M8 | CD79B_M8_v1 | CTCTCCTTACCTCTTAGGTGTGATCTTCC | 52 |
| M8 | CD79B_M8_v2-1 | CTTACCTCTTAGGTGTGATCTTC | 53 |
| M8 | CD79B_M8_v2-2 | CCTTACCTCTTAGGTGTGATC | 54 |
| M8 | CD79B_M8_v2-3 | CCTTACCTCTTAGGTGTGATCT | 55 |
| M8 | CD79B_M8_v3-1 | CCTTACCTCTTAGGTGTGATCTT | 56 |
| M8 | CD79B_M8_v3-2 | TCTCCTTACCTCTTAGGTGTG | 57 |
| M8 | CD79B_M8_v3-3 | CCTTACCTCTTAGGTGTG | 58 |
| M8 | CD79B_M8_v3-4 | TCTCCTTACCTCTTAGGT | 59 |
| M8 | CD79B_M8_v3-5 | TCCTTACCTCTTAGGTGT | 60 |
| M8 | CD79B_M8_v3-6 | TTACCTCTTAGGTGTGATC | 61 |
| M8 | CD79B_M8_v4-1 | TTACCTCTTAGGTGTGAT | 62 |
| M8 | CD79B_M8_v4-2 | TTACCTCTTAGGTGTGA | 63 |
| M8 | CD79B_M8_v4-3 | TCCTTACCTCTTAGGTGTG | 64 |
| M8 | CD79B_M8_v4-4 | TCTCCTTACCTCTTAGGTGT | 65 |
| M9 | CD79B_M9_1 | TCTCCTTACCTCATAGGTGTG | 66 |
| M9 | CD79B_M9_2 | TTACCTCATAGGTGTGATC | 67 |
| M9 | CD79B_M9_3 | TCCTTACCTCATAGGTGTG | 68 |

Table 7 shows a primer set designed in the present Example for amplifying a region comprising the gene mutations in the MYD88 gene.

**[Table 7]**

| Name | Base sequence [5'→3'] | Tm (°C) | SEQ ID NO: |
|---|---|---|---|
| MYD_F01 | ATGCCAGCATGGCACCCCTT | 62.8 | 69 |
| MYD_R01 | GCAGGGGTTGGTGTAGTCGCA | 62.1 | 70 |
| MYD88_Fw-2 | GCAAGGGCCTGATG | 55.6 | 71 |
| MYD88_Rv-2-2 | GTGATGAACCTCAGGATG | 56.8 | 72 |
| MYD88_Fw-3 | GGCTTGTCCCACCAT | 57.9 | 73 |
| MYD88_Rv-3 | TCGCAGACAGTGATGAA | 57.9 | 74 |
| MYD88_Fw-4 | CCTTGGCTTGCAGGT | 58.6 | 75 |
| MYD88_Rv-4 | GGTGTAGTCGCAGACAG | 58.6 | 76 |
| MYD88_Fw-5 | CCTTGGCTTGCAGGTG | 60.0 | 77 |
| MYD88_Rv-5 | CGCAGACAGTGATGAACC | 60.1 | 78 |
| MYD88_Fw-5-3 | CAGGTGCCCATCAGAAG | 59 | 79 |
| MYD88_Rv-5-4 | GATGCTGGGGAACTCTTTCTTCATTGC | 59.9 | 80 |

Table 8 shows a primer set designed in the present Example for amplifying a region comprising the gene mutations in the CD79B gene.

**[Table 8]**

| Name | Base sequence [5'→3'] | Tm (°C) | SEQ ID NO: |
|---|---|---|---|
| CD79B-Fw_1 | ACTCTGATCTCCATCCCTCTC | 62 | 81 |
| CD79B-Rv_1 | CAACCACACCAGCAGATAGT | 62 | 82 |
| CD79B-Fw_3 | CACTCTGATCTCCATCCC | 58.1 | 83 |
| CD79B-Rv_3 | ACCACACCAGCAGATAG | 57.9 | 84 |
| CD79B-Fw_4 | CAGGATGACAGCAAGGC | 59.8 | 85 |
| CD79B-Rv_4 | CAACCACACCAGCAGATAG | 59.9 | 86 |

Table 9 shows blocking nucleic acids corresponding to a wild type for the gene mutations in the MYD88 gene, designed in the present Example.

**[Table 9]**

| Name | Base sequence [5'→3'] | SEQ ID NO: |
|---|---|---|
| MYD88_B_v1 | GCGACTGATCCCCA | 87 |
| MYD88_B_v2-1 | GCGACTGATCCCCAT | 88 |
| MYD88_B_v2-2 | GACTGATCCCCATC | 89 |
| MYD88_B_v2-3 | AGAAGCGACTGATC | 90 |

Table 10 shows blocking nucleic acids corresponding to a wild type for the gene mutations in the CD79B gene, designed in the present Example.

**[Table 10]**

| Name | Base sequence [5'→3'] | SEQ ID NO: |
|---|---|---|
| CD79B_B_v1 | ACCTCGTAGGTGTGA | 91 |
| CD79B_B_v2-3 | TTACCTCGTAGGTGT | 92 |
| CD79B_B_v3-3 | CCTTACCTCGTAGGT | 93 |
| CD79B_B_v4-1 | CTTACCTCGTAGGTGTG | 94 |
| CD79B_B_v4-2 | TACCTCGTAGGTGTGA | 95 |
| CD79B_B_v4-3 | TCCTTACCTCGTAGGTG | 96 |

### <Study of Optimal Mutant-Type Probes>

For the probes described in Tables 4 to 6, fluorescence intensity was measured using a 100% mutation model specimen or a 5% mutation model specimen. Specifically, a DNA chip on which the above various probes had been immobilized was set in a genetic analyzer BIOSHOT HT-32 (manufactured by Toyo Kohan Co., Ltd.), and hybridization reaction with the model specimen was carried out. A DNA chip needle on which the DNA chip had been fixed, and further, a washing solution (0.1×SSC/0.1% SDS solution, room temperature) for washing away excess reagents after the hybridization reaction of the DNA chip, a rinse solution (1×SSC solution, room temperature), and a detection solution for detecting fluorescence (1×SSC solution, room temperature) were set in BIOSHOT HT-32, and operation was started.

By automatic operation, the DNA chip needle was inserted into a designated PCR tube containing a reaction solution heated at 51.5°C, and hybridization reaction was allowed to proceed for 30 minutes. After the hybridization reaction, the DNA chip needle was quickly immersed in a washing solution tank, and stirred 30 times such that the DNA chip moved in and out of the surface of the washing solution. The washed DNA chip needle was immersed in a rinse solution tank, and stirred 80 times such that the DNA chip moved in and out of the surface of the rinse solution.

The rinsed DNA chip needle was slowly immersed in a detection solution tank, and irradiated with a 640 nm single-wavelength laser to capture excitation light with a CCD camera for 2 seconds. Thereafter, the value for determination was calculated from the measured fluorescence intensity value according to the following formula. 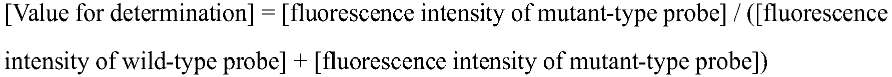 [Value for determination] = [fluorescence intensity of mutant-type probe] / ([fluorescence intensity of wild-type probe] + [fluorescence intensity of mutant-type probe])

The "100% mutation model specimen" means a sample in which the ratio between a mutant type and a wild type is 100:0 for the gene mutations to be detected. The "5% mutation model specimen" means a sample in which the ratio between a mutant type and a wild type is 5:95 for the gene mutations to be detected.

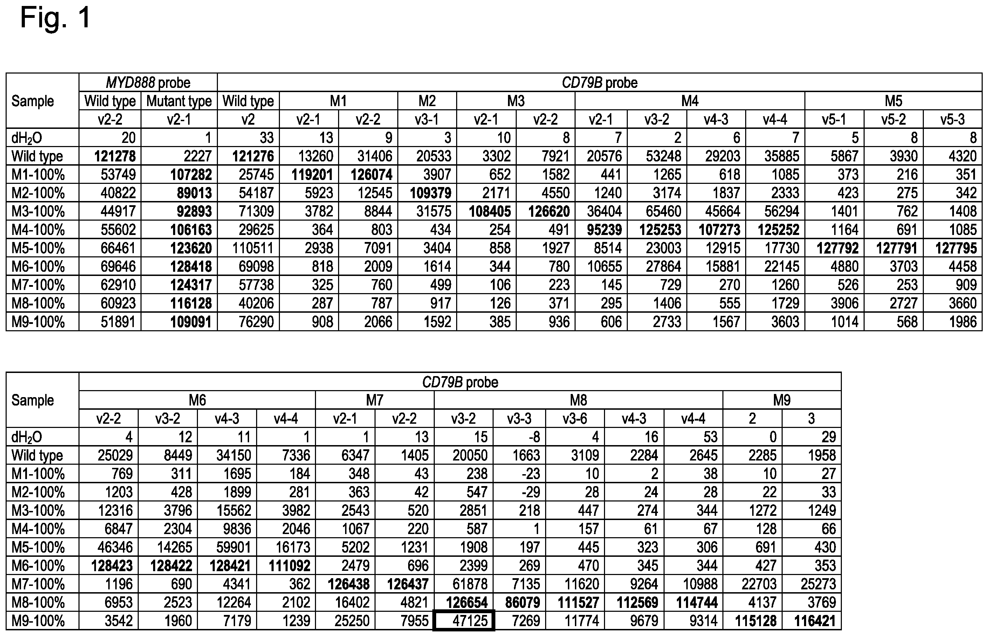

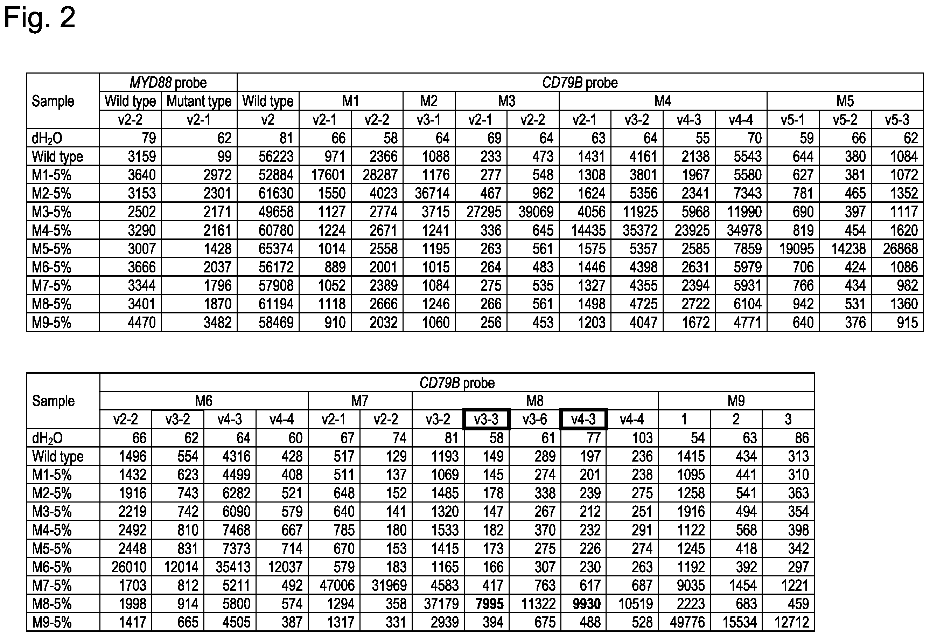

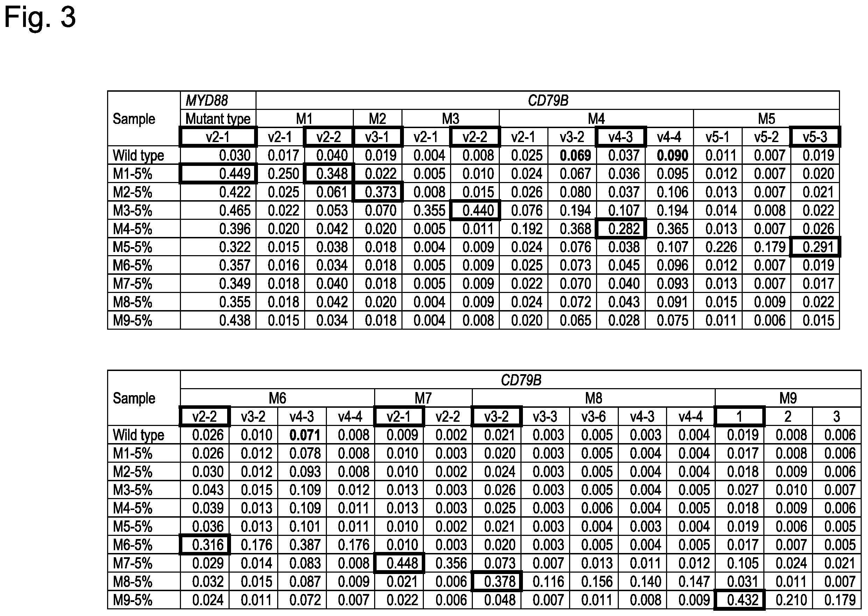

Fig. 1 shows the results of measurement of fluorescence intensity values in the cases where the 100% mutation model specimen was used; Fig. 2 shows the results of measurement of fluorescence intensity values in the cases where the 5% mutation model specimen was used; and Fig. 3 shows the results of measurement of values for determination from the results shown in Fig. 2. For comparison, Figs. 1 to 3 also show the results of calculation of the fluorescence intensity value from each probe using a wild-type sample, in which the ratio between a mutant type and a wild type is 0:100 for the gene mutations to be detected.

From the fluorescence intensity values shown in Fig. 1, specificity of the mutant-type probes and the wild-type probes was investigated. As a result, it was found that excellent fluorescence intensity values can be obtained for all mutant-type probes and wild-type probes tested. Further, for all mutant-type probes and wild-type probes tested, cross-reactivity was suppressed to about 1/2 or less relative to the specific fluorescence intensity value. Therefore, it could be determined that the target gene mutations (mutant type, wild type) can be distinguished from each other.

Further, according to the results shown in Fig. 1, when the M8 probe (CD79B_M8_v3-2) was used, a strong fluorescence intensity value (47,125) was detected for the silent mutation 591C>T, denoted as M9, among the gene mutations in the CD79B gene. Thus, unless detection of M9, which is a silent mutation, is intended using a mutant-type probe, there is a possibility that a sample having the M9 silent mutation may be erroneously identified as the M8 mutation. Therefore, in identification of the gene mutations in the CD79B gene, erroneous determination can be prevented by using a mutant-type probe corresponding to the silent mutation M9 to securely identify this silent mutation. At this time, when the silent mutation is identified, it is determined as a "wild type." Therefore, a mutant type or a wild type can be precisely identified for the gene mutations in the CD79B gene, and very useful information can be provided for a disease related to the gene mutations in the CD79B gene.

On the other hand, Fig. 2 shows fluorescence intensity values obtained with the mutant-type probes and the wild-type probes in cases where samples having a low mutation ratio were used. Even in the cases of a sample having a low mutation ratio, a target gene mutation can be detected with high accuracy by acquiring a fluorescence intensity value that is not less than a desired value. For example, when a fluorescence intensity value of not less than 10,000 is preferred, the results shown in Fig. 2 indicate that sufficient fluorescence intensity values were not obtained for CD79B_M8_v3-3 and CD79B_M8_v4-3.

Fig. 3 also shows values for determination calculated from the fluorescence intensity values shown in Fig. 2 according to the above formula. As can also be understood from the formula for calculating the value for determination, a higher value for determination indicates a better separation performance achieved with a mutant-type probe and a wild-type probe. It can also be said that, when a wild-type sample is used, the lower the value for determination calculated for a mutant-type probe, the better the separation performance. For example, when a value for determination of not more than 0.05 as calculated for a mutant-type probe using a wild-type sample is used as a criterion, it can be seen from the results shown in Fig. 3 that CD79B_M4_v3-2, CD79B_M4_v4-4, and CD79B_M6-v4-3 do not have a sufficient separation performance.

Taking the above-described results into account as a whole, it was found that MYD88_M_v2-1 can be preferably employed as a mutant-type probe for the gene mutations in the MYD88 gene. It was also found that CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-1 can be preferably employed for the gene mutations (9 types: M1 to M9) in the CD79B gene.

### <Study of Optimal Wild-Type Probes>

First, wild-type probes for detecting a wild type for the gene mutations in the MYD88 gene were studied. Fluorescence intensity values and values for determination from wild-type probes and a mutant-type probe (v2-1) were calculated for cases using a wild-type sample and a 5% mutation model specimen provided for the test. Further, fluorescence intensity values and values for determination were similarly calculated for cases using genomic DNA (gDNA-1) in place of the wild-type sample and the 5% mutation model specimen.

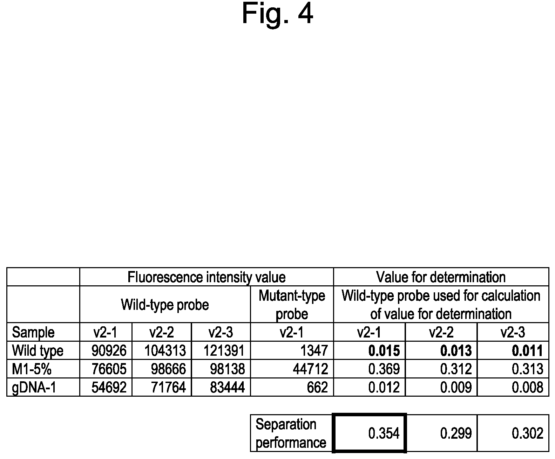

The results are shown in Fig. 4. Selection of a wild-type probe was carried out such that the following requirements were satisfied: the probe shows a high fluorescence intensity value when the genomic DNA is used; the probe has a high separation performance (in other words, there is a large difference in the value for determination between a mutant type and a wild type); and the value for determination calculated according to the above formula from a fluorescence intensity value obtained using the wild-type sample is low. Specifically, the following criteria were provided, and comprehensive evaluation was performed based on these criteria: the fluorescence intensity value from the wild-type probe is not less than 10,000 when the genomic DNA is used; the probe has the highest separation performance among the wild-type probes tested (the probe shows the largest difference in the value for determination between a mutant type and a wild type); and the value for determination is not more than 0.05 when the wild-type sample is used. As a result, v2-1, which had the highest separation performance, could be selected as the optimal wild-type probe.

Subsequently, wild-type probes for detecting a wild type for the gene mutations in the CD79B gene were studied. The study method and the evaluation criteria were the same as those in the above-described method for selecting a wild-type probe for the gene mutations in the MYD88 gene. As mutant-type probes, CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-1, which were selected as described above, were used.

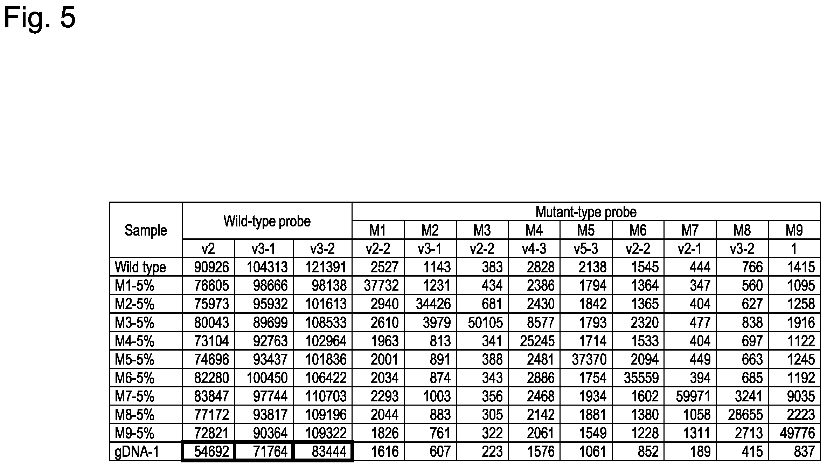

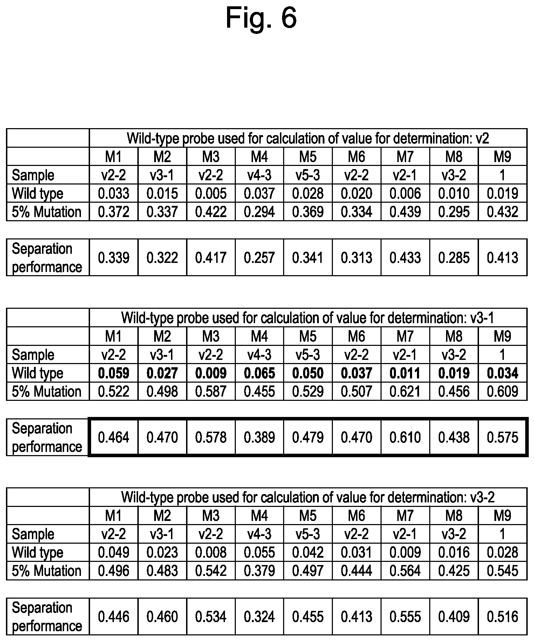

The results of measurement of the fluorescence intensity value are shown in Fig. 5, and the results of calculation of the value for determination from the fluorescence intensity value are shown in Fig. 6. As can be seen from Figs. 5 and 6, v3-1 could be selected as the optimal wild-type probe taking into account the following facts as a whole: the probe showed a high fluorescence intensity value when genomic DNA was used; the probe had a high separation performance; and the value for determination was low when the wild-type sample was used.

### <Evaluation of Performances of Selected Mutant-Type Probes and Wild-Type Probes>

Using the mutant-type probe (MYD88_M_v2-1) and the wild-type probe (MYD88_W_v2-1) selected for the gene mutations in the MYD88 gene, and the mutant-type probes (CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-1) and the wild-type probe (CD79B_W_v3-1) selected for the gene mutations in the CD79B gene, those gene mutations in genomic DNA were identified. The following three types of genomic DNA were used: gDNA-1, gDNA-2, and gDNA-3. gDNA-1 is genomic DNA derived from a healthy individual. gDNA-2 is genomic DNA derived from a patient with non-Hodgkin lymphoma. gDNA-3 is genomic DNA derived from lymphoplasmacytic lymphoma.

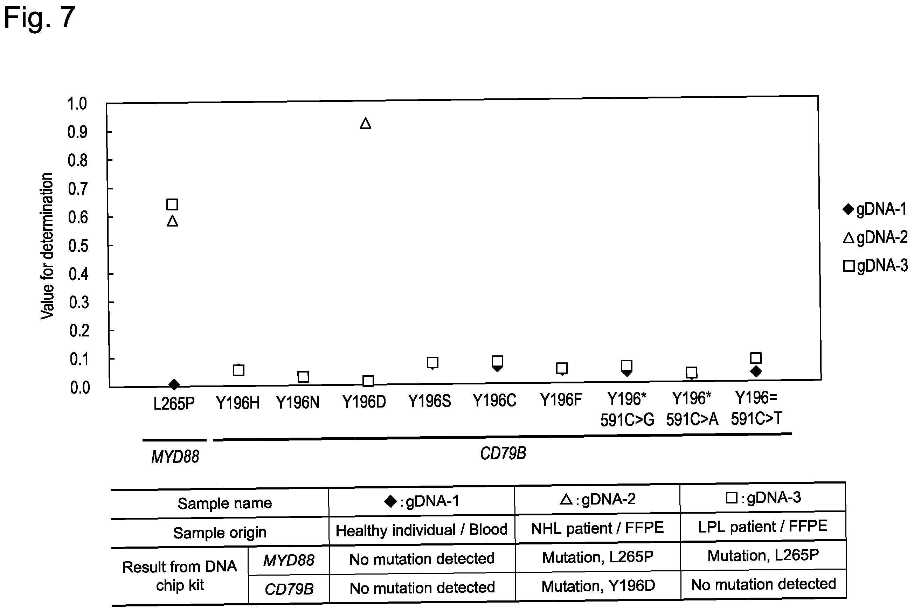

The results of identification of each gene mutation using these mutant-type probes and wild-type probes are shown in Fig. 7. As shown in Fig. 7, gDNA-1 was identified as not being a mutant type for the gene mutations in the MYD88 gene, and not being a mutant type for the gene mutations in the CD79B gene. Further, gDNA-2 was identified as being a mutant type (L265P) for the gene mutations in the MYD88 gene, and being a mutant type (Y196D, M3) for the gene mutations in the CD79B gene. Further, gDNA-3 was identified as being a mutant type (L265P) for the gene mutations in the MYD88 gene, and not being a mutant type for the gene mutations in the CD79B gene.

In order to verify the results obtained using the mutant-type probes and the wild-type probes shown in Fig. 7, the three types of genomic DNA tested (gDNA-1, gDNA-2, and gDNA-3) were subjected to the direct sequencing method to confirm the gene mutations in the MYD88 gene and the CD79B gene. As a result, gDNA-1 could be confirmed to be a wild type for both the gene mutations in the MYD88 gene and the gene mutations in the CD79B gene. Further, gDNA-1 could be confirmed to be the mutant type L265P for the gene mutations in the MYD88 gene, and the mutant type Y196D (M3) for the gene mutations in the CD79B gene. Further, gDNA-3 could be confirmed to be the mutant type L265P for the gene mutations in the MYD88 gene, and a wild type for the gene mutations in the CD79B gene.

The results of identification of the gene mutations using the mutant-type probes and wild-type probes selected as described above were completely consistent with the results confirmed by the direct sequencing method. It was thus shown that the mutant-type probes and the wild-type probes selected as described above can accurately detect the gene mutations with high accuracy even in practical situations.

### <Design of Primer Sets>

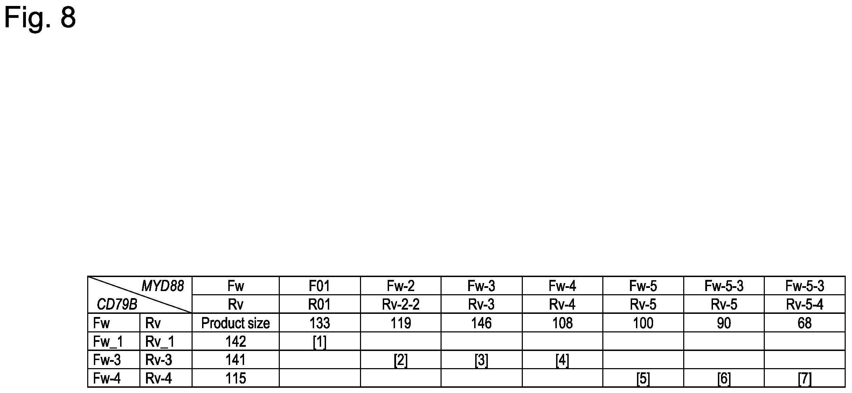

For the primer set for amplifying a region comprising the gene mutations in the MYD88 gene and the primer set for amplifying a region comprising the gene mutations in the CD79B gene shown in Tables 7 and 8, the seven combinations ([1] to [7]) shown in Fig. 8 were tested, and optimal primer sets were selected on the basis of the amount of amplification of a target nucleic acid fragment and the amount of amplification of non-specific nucleic acid.

First, a reaction solution was prepared using a wild-type model specimen or genomic DNA (gDNA) as a template. Using the prepared PCR reaction solution, PCR was performed by thermal cycling as follows: 95°C for 5 minutes, followed by cycles of [95°C for 30 seconds, a predetermined annealing temperature (for example, 59°C) for 30 seconds, and 72°C for 45 seconds]. Thereafter, the temperature was kept at 72°C for 10 minutes, and then maintained at 4°C. The annealing temperature was 56°C, 58°C, 60°C, 62°C, or 64°C.

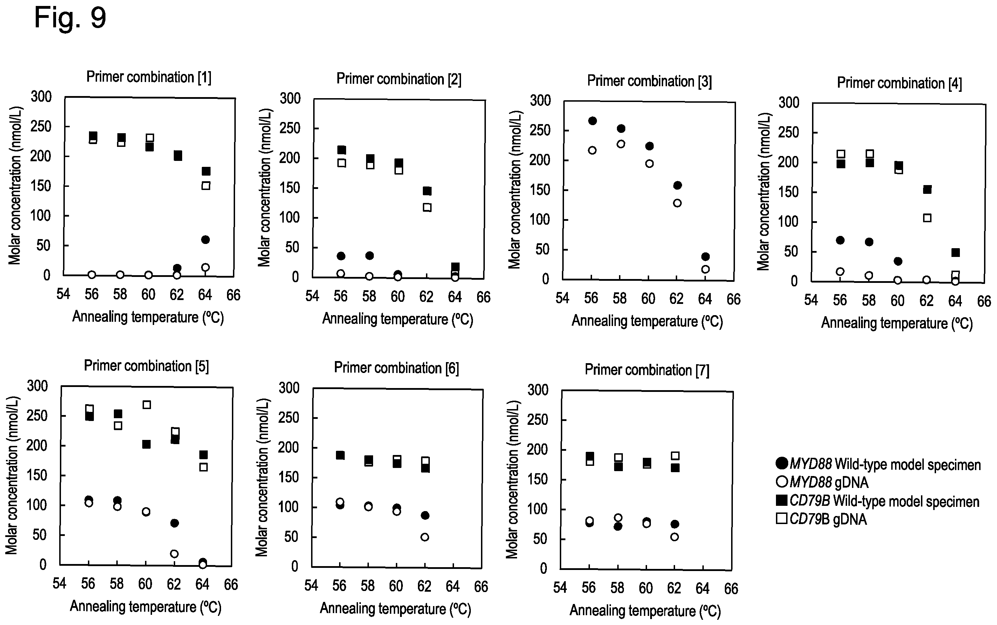

Fig. 9 shows the relationship between the amount of amplification of nucleic acid and the annealing temperature. As can be seen from Fig. 9, the combinations [5], [6], and [7] were capable of amplifying a region comprising the gene mutations in the MYD88 gene and a region comprising the gene mutations in the CD79B gene from both the wild-type model specimen and the genomic DNA to the same level (capable of multiplex amplification).

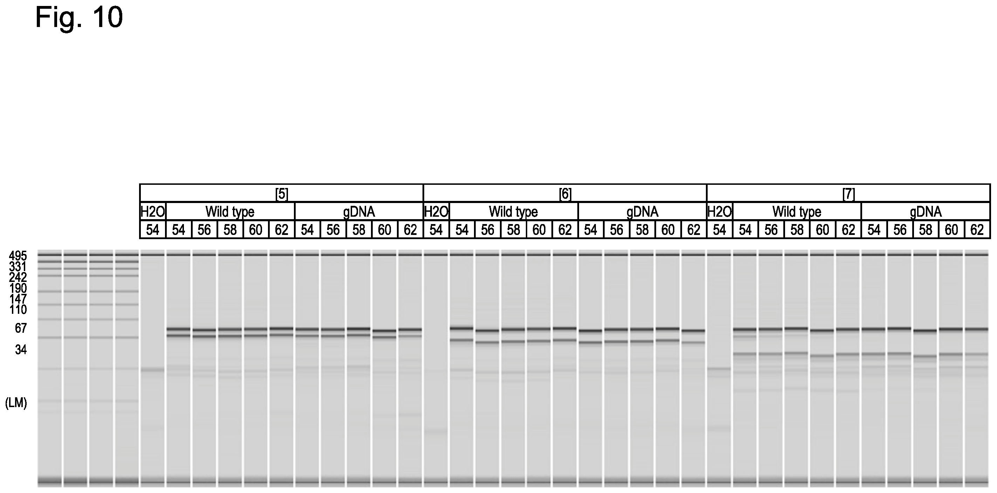

Fig. 10 shows the results of confirming, by electrophoresis, nucleic acid fragments comprised in the reaction solutions after PCR performed with the combinations [5], [6], and [7]. As shown in Fig. 10, when the combination [7] was used, a plurality of nucleic acid fragments due to non-specific amplification, which are fragments other than the target nucleic acid fragments, could be confirmed. From these results, the combinations [5] and [6] were confirmed to be superior to the combination [7].

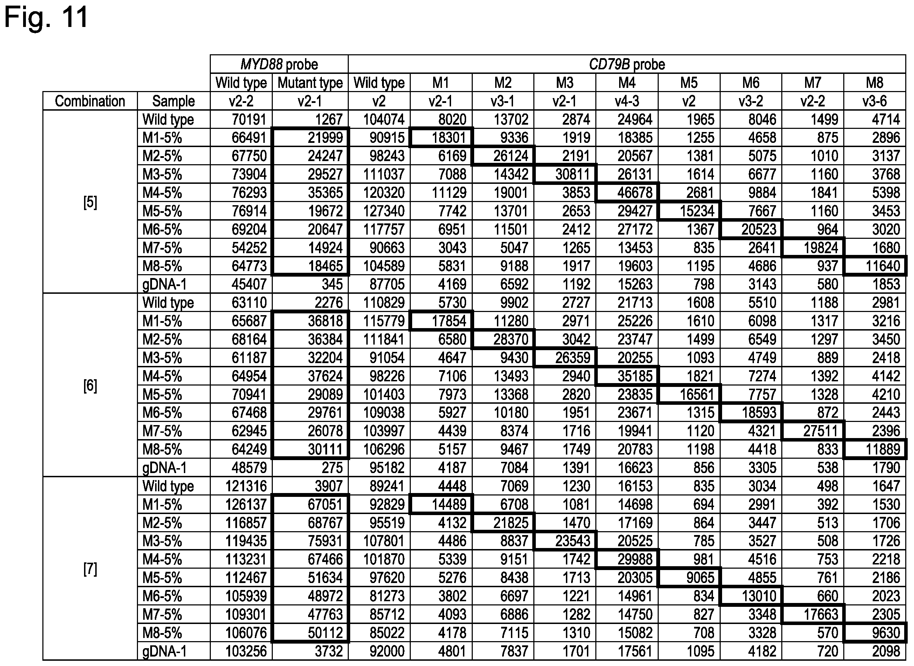

Subsequently, a reaction solution was prepared using a 5% mutation model specimen or genomic DNA derived from a healthy individual (gDNA-1) as a template. PCR was performed in the same manner using the reaction solution, and fluorescence intensity based on the amplified fragments was measured. The results are shown in Fig. 11. As shown in Fig. 11, when the combination [5] was used, the fluorescence intensity from the mutant-type probe corresponding to the mutant type for the gene mutations in the MYD88 gene was found to be low as compared with the other combinations.

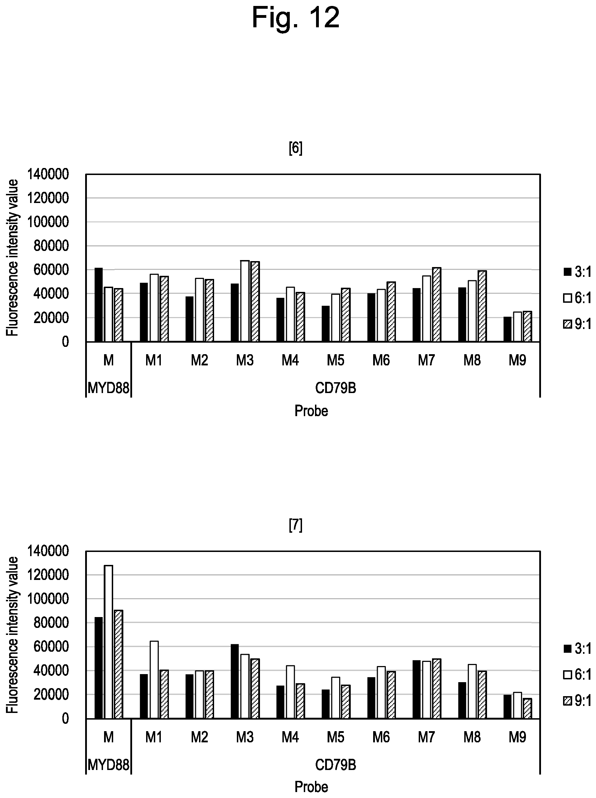

For the combinations [6] and [7], changes in the fluorescence intensity value were studied while changing the mixing ratio between a labeled primer and an unlabeled primer. The results are shown in Fig. 12. As shown in Fig. 12, in the case of the combination [6], the fluorescence intensity increased as the mixing ratio of the labeled primer increased, and the fluorescence intensity value obtained was in accordance with the ratio of the labeled primer. In contrast, in the case of the combination [7], different tendencies of change were found between the ratio of the labeled primer and the fluorescence intensity value. This was considered to be due to the non-specific amplification observed when the combination [7] was used. From these results, it became clear that the combination [6] can be preferably used for multiplex amplification of a region comprising the gene mutations in the MYD88 gene and a region comprising the gene mutations in the CD79B gene.

### <Study of Primer Ratios (Labeled vs Unlabeled)>

For each of a primer set for amplifying a region comprising the gene mutations in the MYD88 gene and a primer set for amplifying a region comprising the gene mutations in the CD79B gene, the optimal ratio between a labeled primer and an unlabeled primer was studied by changing the ratio between the labeled primer and the unlabeled primer while investigating how the fluorescence intensity value changed. In the present Example, in the primer set for amplifying a region comprising the gene mutations in the CD79B gene, the forward primer was a labeled primer (CD79B_Fw-4), and the reverse primer was an unlabeled primer (CD79B_Rv-4). Further, in the primer set for amplifying a region comprising the gene mutations in the MYD88 gene, the forward primer was an unlabeled primer (MYD88_Fw-5-3), and the reverse primer was a labeled primer (MYD88_Rv-5).

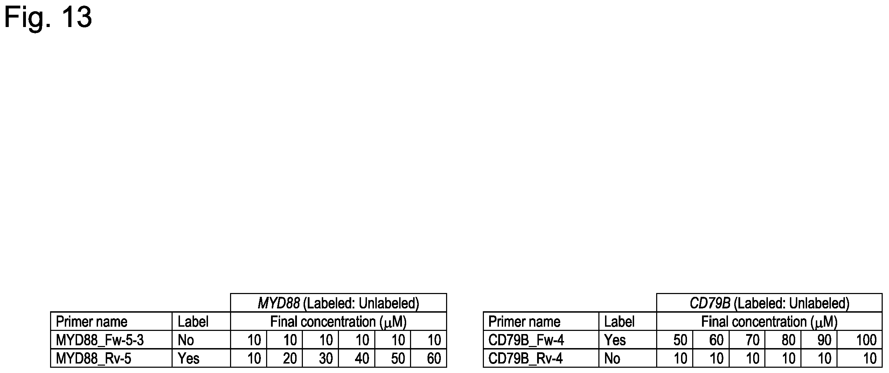

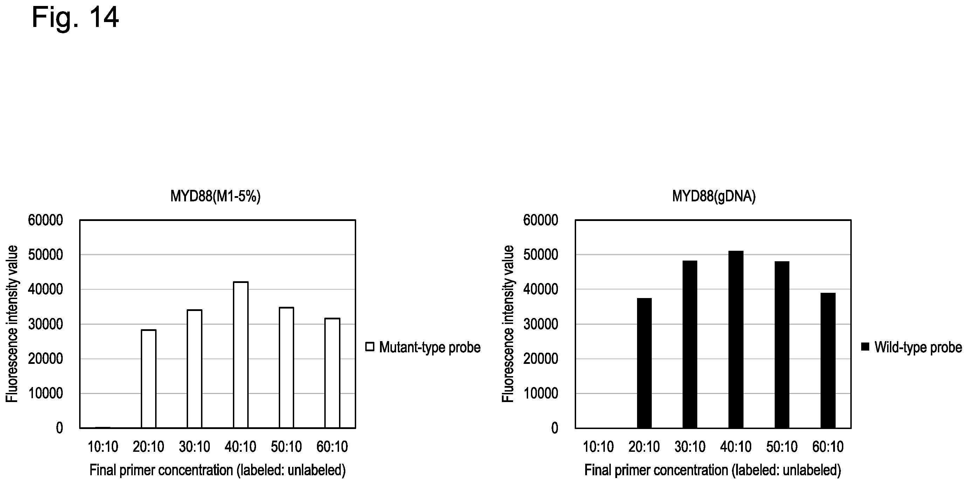

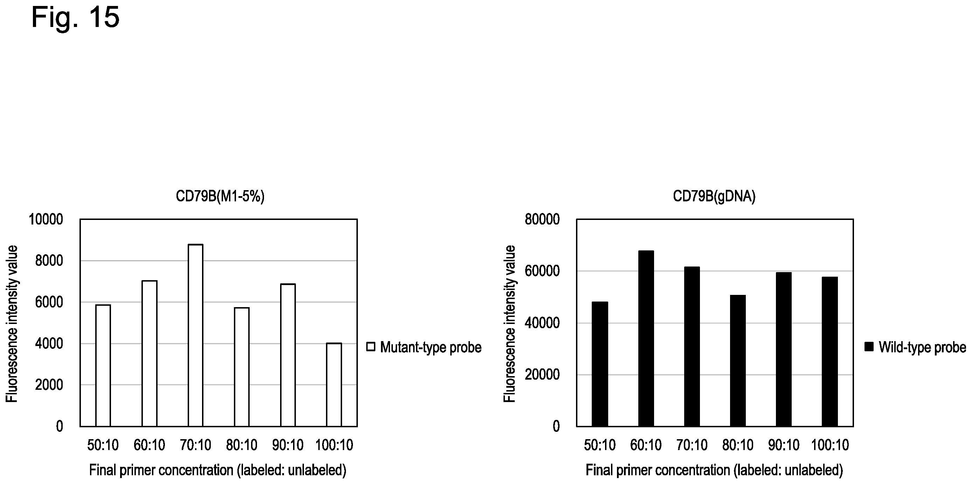

In the present Example, as shown in Fig. 13, the final concentrations of the labeled primers (CD79B_Fw-4, MYD88_Rv-5) and the unlabeled primers (CD79B_Rv-4, MYD88_Fw-5-3) comprised in the PCR reaction solution were adjusted, and PCR was performed in the same manner. A 5% mutation model specimen and genomic DNA were used as templates. The results obtained using the primer set for amplifying a region comprising the gene mutations in the MYD88 gene are shown in Fig. 14, and the results obtained using the primer set for amplifying a region comprising the gene mutations in the CD79B gene are shown in Fig. 15.

As can be seen from Fig. 14, when the final concentration of the labeled primer for amplifying a region comprising the gene mutations in the MYD88 gene was within the range of 2 times (that is, labeled primer: unlabeled primer = 20:10) to 6 times that of the unlabeled primer, high fluorescence intensity values were achieved. In particular, higher fluorescence intensity values were achieved within the range of 3 times to 5 times, and the highest fluorescence intensity value could be achieved at 4 times.

Further, as can be seen from Fig. 15, when the final concentration of the labeled primer for amplifying a region comprising the gene mutations in the CD79B gene was within the range of 5 times to 10 times that of the unlabeled primer, high fluorescence intensity values were achieved. In particular, higher fluorescence intensity values were achieved within the range of 6 times to 7 times, and the highest fluorescence intensity value could be achieved at 7 times.

### <Study of Optimal Blocking Nucleic Acids>

From the blocking nucleic acids for the gene mutations in the MYD88 gene shown in Table 9 and the blocking nucleic acids for the gene mutations in the CD79B gene shown in Table 10, optimal blocking nucleic acids were selected based on the function as a blocking nucleic acid. A blocking nucleic acid has a function of specifically hybridizing with a wild-type nucleic acid fragment among nucleic acid fragments obtained by amplifying a region comprising gene mutations, and this function prevents the wild-type nucleic acid fragment from non-specifically hybridizing with a mutant-type probe. Therefore, in selecting an optimal blocking nucleic acid, non-specific hybridization between the wild-type nucleic acid fragment and the mutant-type probe in the presence of the blocking nucleic acid can be determined based on the fluorescence intensity of the mutant-type probe. Further, whether the blocking nucleic acid inhibits specific hybridization between the mutant-type nucleic acid fragment and the mutant-type probe in the presence of the blocking nucleic acid can be determined based on the fluorescence intensity of the mutant-type probe.

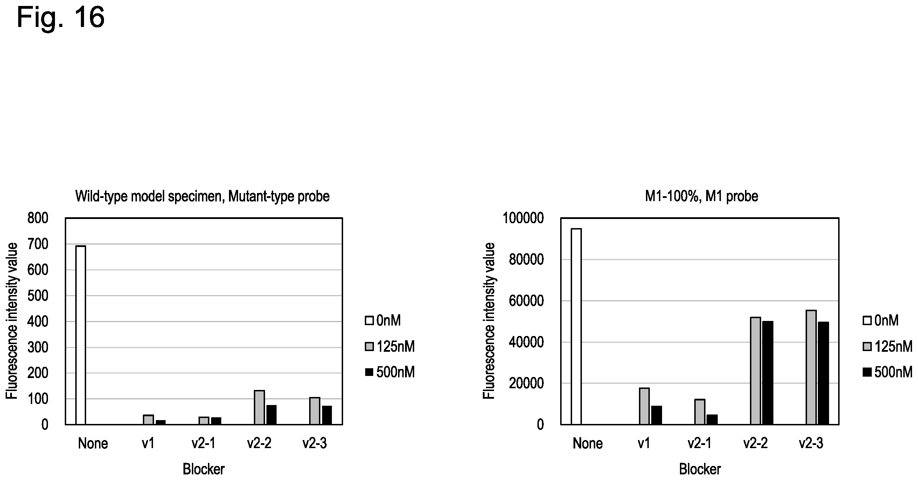

In the presence of each blocking nucleic acid (125 nM or 500 nM) for the gene mutations in the MYD88 gene shown in Table 9, hybridization was carried out between a wild-type model specimen and a mutant-type probe (MYD88_M_v2-1), and the fluorescence intensity of the mutant-type probe was measured. In addition, in the presence of each blocking nucleic acid, hybridization was carried out between a 100% mutation model specimen and the mutant-type probe (MYD88_M_v2-1), and the fluorescence intensity of the mutant-type probe was measured. The results are shown in Fig. 16.

As can be seen from Fig. 16, non-specific hybridization between the wild-type model specimen and the mutant-type probe could be suppressed for all of the blocking nucleic acids tested. However, among the blocking nucleic acids, v1 and v2-1 were found to inhibit specific hybridization between the 100% mutation model specimen and the mutant-type probe. In view of this, using a 5% mutation model specimen, v2-2 and v2-3 were examined to see which functions as a better blocking nucleic acid.

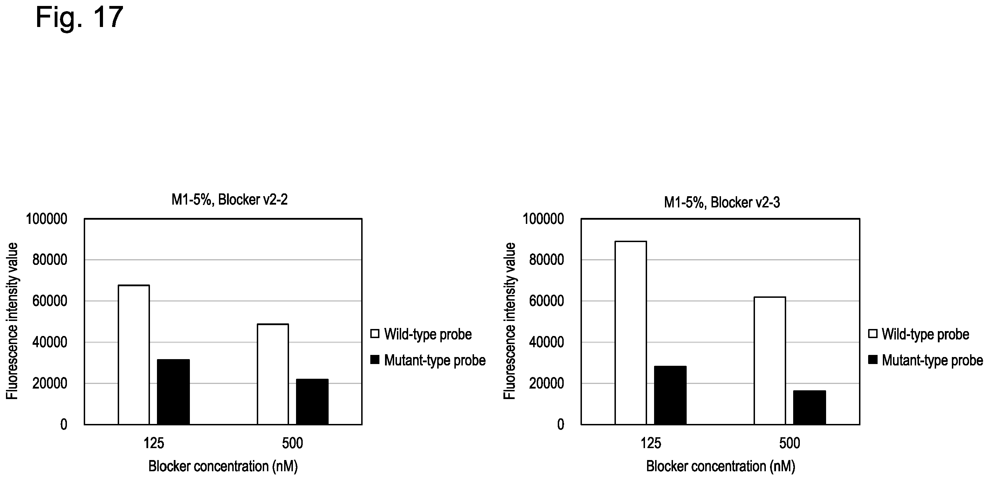

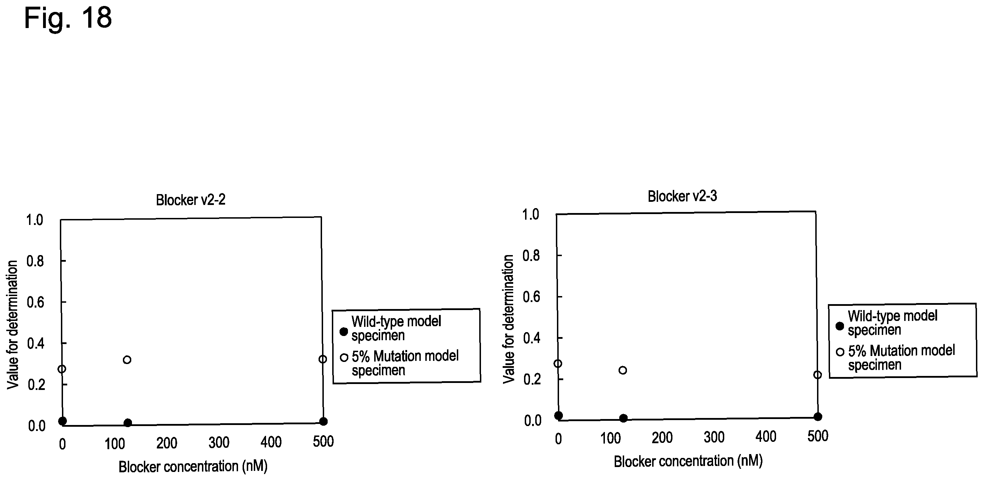

Specifically, in the presence of v2-2 or v2-3 (125 nM or 500 nM), hybridization was carried out between a 5% mutation model specimen and both a wild-type probe (MYD88_W_v2-1) and a mutant-type probe (MYD88_M_v2-1), and the fluorescence intensities of the wild-type probe and the mutant-type probe were measured. Further, values for determination were calculated from the measured fluorescence intensity values according to the above formula. The results of measurement of the fluorescence intensities are shown in Fig. 17, and the results of calculation of the values for determination are shown in Fig. 18.

As can be seen from Figs. 17 and 18, in the cases where v2-2 was used, the fluorescence intensity of the wild-type probe was lower than in the cases where v2-3 was used, indicating that v2-2 functions better as a blocking nucleic acid compared with v2-3. Based on the results, among the blocking nucleic acids shown in Table 9, v2-2 was selected as the optimal blocking nucleic acid for the gene mutations in the MYD88 gene. These results also indicated that, even without use of a blocking nucleic acid for the gene mutations in the MYD88 gene (that is, even at 0 nM), the wild-type model specimen and the 5% mutation model specimen show sufficiently separated values for determination. From the above results, it was found that the concentration of the blocking nucleic acid for the gene mutations in the MYD88 gene is preferably 0 to 500 nM.

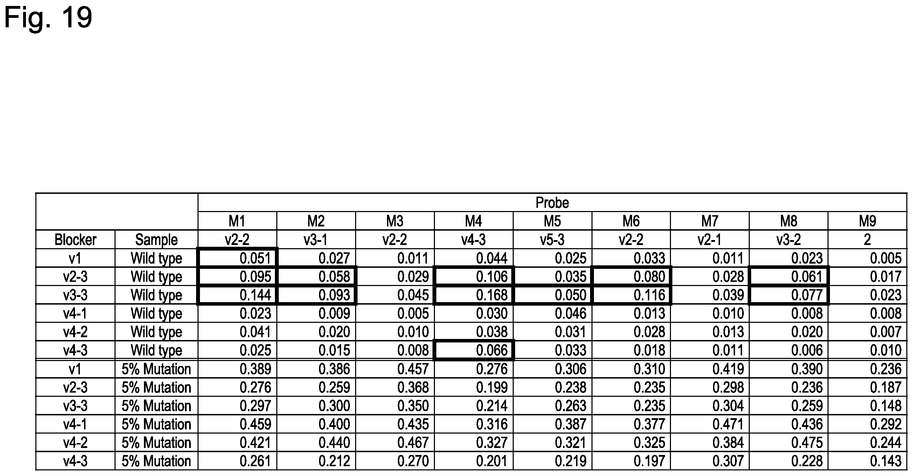

Subsequently, in the presence of each blocking nucleic acid for the gene mutations in the CD79B gene shown in Table 10, hybridization was carried out between a wild-type model specimen or a 5% mutation model specimen, and a wild-type probe (CD79B_W_v3-1) and mutant-type probes (CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-2), and the fluorescence intensities from the wild-type probe and the mutant-type probes were measured, followed by calculating values for determination according to the above formula. The results are shown in Fig. 19.

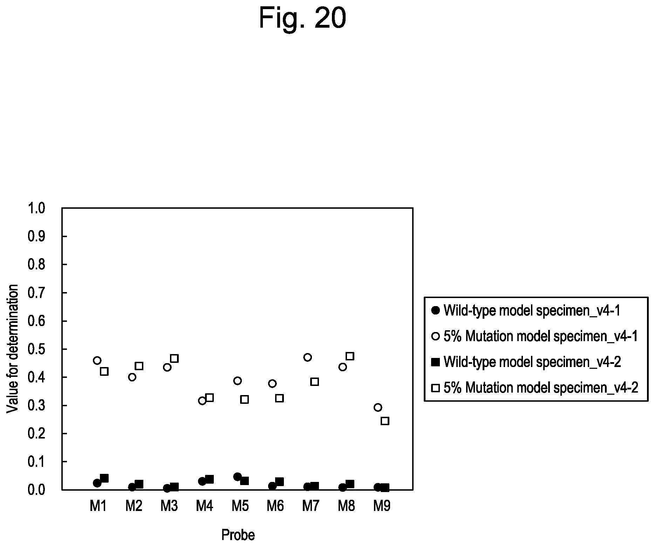

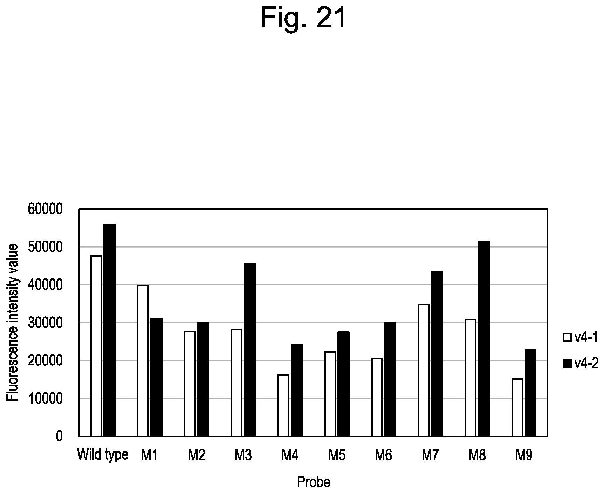

As shown in Fig. 19, when the wild-type model specimen was used, the blocking nucleic acids v4-1 and v4-2 were the only blocking nucleic acids with which all mutant-type probes showed values for determination of not more than 0.05. Figs. 20 and 21 show the values for determination and the fluorescence intensity values, respectively, of each mutant-type probe as calculated for the blocking nucleic acids v4-1 and v4-2 using the wild-type model specimen or the 5% mutation model specimen. As can be seen from Figs. 20 and 21, v4-1 and v4-2 were almost equivalent based on comparison of the value for determination, whereas v4-2 showed higher values based on comparison of the fluorescence intensity value between v4-1 and v4-2. Based on these results, among the blocking nucleic acids shown in Table 10, v4-2 was selected as the optimal blocking nucleic acid for the gene mutations in the CD79B gene.

### <Study of Blocking Nucleic Acid Concentration>

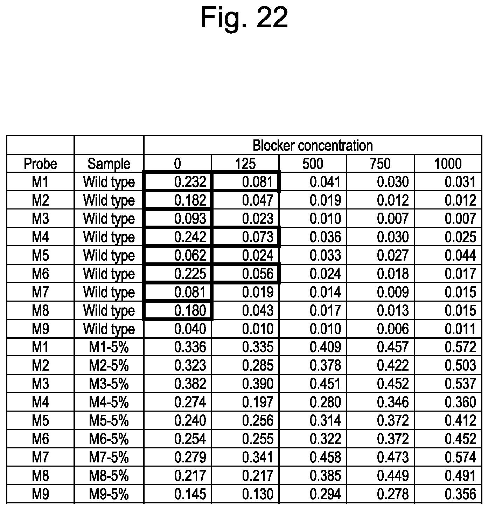

In the presence, at various concentrations, of v4-2, which was selected as the blocking nucleic acid for the gene mutations in the CD79B gene, hybridization was carried out between a wild-type model specimen or a 5% mutation model specimen, and a wild-type probe (CD79B_W_v3-1) and mutant-type probes (CD79B_M1_v2-2, CD79B_M2_v3-1, CD79B_M3-v2-2, CD79B_M4_v4-3, CD79B_M5_v5-3, CD79B_M6_v2-2, CD79B_M7_v2-1, CD79B_M8_v3-2, and CD79B_M9-2), and the fluorescence intensities from the wild-type probe and the mutant-type probes were measured, followed by calculating values for determination according to the above formula. The results are shown in Fig. 22.

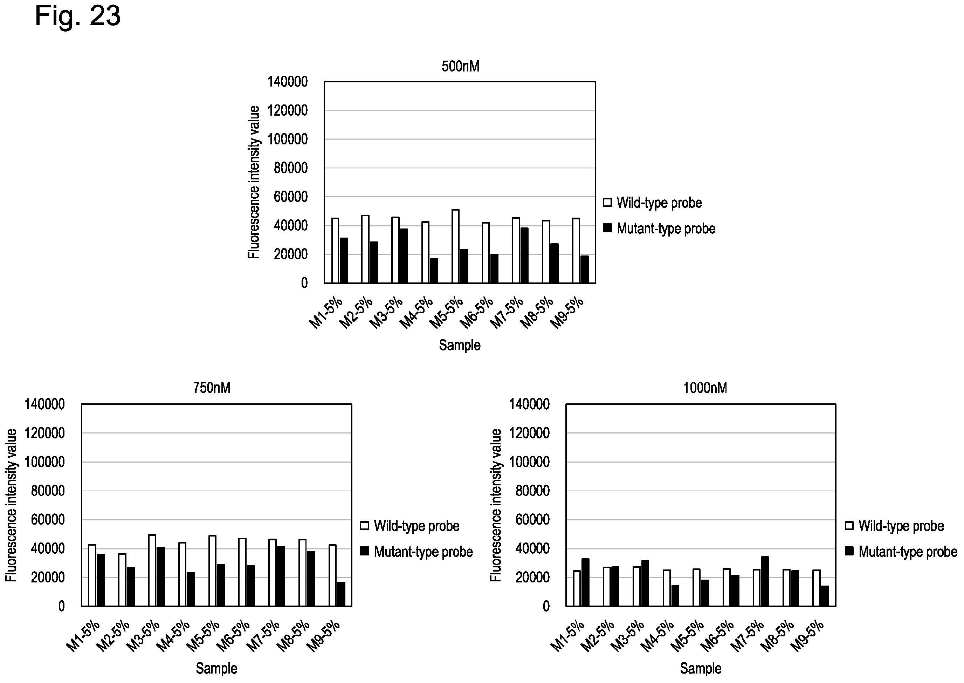

As shown in Fig. 22, in the cases where the concentration of the blocking nucleic acid was 125 nM, a plurality of mutant-type probes showed values for determination exceeding 0.05 when the wild-type model specimen was used. These results indicate that the concentration of the blocking nucleic acid is preferably higher than 125 nM. Fig. 23 shows the results of measurement of the fluorescence intensity from each mutant-type probe in cases where the concentration of the blocking nucleic acid was 500 nM, 750 nM, or 1000 nM, and where the 5% mutation model specimen was used. From these results, it was found that a blocking nucleic acid concentration of 1000 nM results in a lower fluorescence intensity value compared to those in the cases where the concentration is 500 nM or 750 nM. These results suggest that the concentration of the blocking nucleic acid is preferably 500 nM or 750 nM.

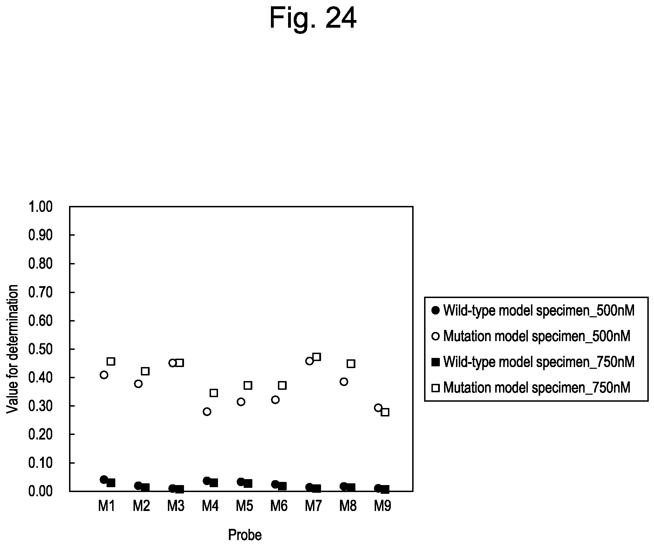

In order to confirm the above results, the value for determination of each mutant-type probe was calculated for the cases where the concentration of the blocking nucleic acid was 500 nM or 750 nM, and where the wild-type model specimen or the 5% mutation model specimen was used. The results are shown in Fig. 24. As can be seen from Fig. 24, in both cases where the concentration of the blocking nucleic acid was 500 nM or 750 nM, the wild-type model specimen and the 5% mutation model specimen showed sufficiently separated values for determination, indicating that the wild type and each mutant type can be accurately detected. In particular, when the concentration of the blocking nucleic acid was 750 nM, the wild-type model specimen and the 5% mutation model specimen showed values for determination that are separated from each other to a greater extent compared to those in the cases where the concentration was 500 nM.

From the above results, it was found that the concentration of the blocking nucleic acid can be within the range of 125 nM to 1000 nM, and that the concentration is preferably 500 to 750 nM. For example, the concentration of the blocking nucleic acid was found to be most preferably 750 nM.

All publications, patents, and patent applications cited in the present description are incorporated into the present description by reference in their entirety.

## Claims

1. A method for detecting gene mutations comprising a missense mutation and/or a nonsense mutation, and a silent mutation, comprising:
amplifying a nucleic acid region comprising the gene mutations;
hybridizing a nucleic acid fragment comprising the gene mutations with a first probe corresponding to the missense mutation or the nonsense mutation in the gene mutations, a second probe corresponding to the silent mutation in the gene mutations, and a wild-type probe corresponding to a wild type; and
detecting signals from the first probe, the second probe, and the wild-type probe,
wherein the signal from the second probe is determined as a wild type for the gene mutations.

2. The method of claim 1, wherein a value for determination is calculated using the following formula: value for determination = [signal value from the second probe] / [signal value from the wild-type probe + signal value from the second probe], and a wild type is determined for the gene mutations when the calculated value for determination is more than a predetermined cutoff value.

3. The method of claim 1, wherein a value for determination is calculated using the following formula: value for determination = [signal value from the first probe] / [signal value from the wild-type probe + signal value from the first probe], and the presence of the missense mutation and/or the nonsense mutation is determined for the gene mutations when the calculated value for determination is more than a predetermined cutoff value.

4. The method of claim 3, wherein value for determinations are calculated using the formula for each of a plurality of the missense mutation and/or the nonsense mutation in the gene mutations, and the presence of each of the plurality of the missense mutation and/or nonsense mutation is determined.

5. The method of claim 4, wherein a wild type is determined for the gene mutations when all of the values for determination calculated for each of the plurality of the missense mutation and/or nonsense mutation are less than the cutoff value.

6. The method of claim 1, wherein the gene mutations are a missense mutation and/or a nonsense mutation occurring in a codon encoding a particular amino acid, and the first probe corresponds to a missense mutation and/or a nonsense mutation having a base substitution at the same position as a base substitution that results in the silent mutation for the amino acid.

7. The method of claim 1, wherein the gene mutations are a missense mutation and/or a nonsense mutation occurring in a codon encoding a particular amino acid, and the first probe corresponds to a missense mutation and/or a nonsense mutation having a base substitution at a codon in which a base substitution that results in a silent mutation for the amino acid occurs.

8. The method of claim 7, comprising using a plurality of first probes for all missense mutations and/or nonsense mutations having a base substitution in a codon in which a base substitution resulting in a silent mutation for the above amino acid occurs.

9. The method of claim 1, wherein the gene mutations comprise a missense mutation and/or a nonsense mutation for the tyrosine residue at position 196 and a silent mutation for the tyrosine residue in the CD79B gene.

10. The method of claim 9, wherein the missense mutation in the CD79B gene is at least one mutation selected from the group consisting of 589T>C, 589T>A, 589T>G, 590A>C, 590A>G and 590A>T, the nonsense mutation in the CD79B gene is 591C>G or 591C>A, and the silent mutation in the CD79B gene is 591C>T.

11. The method of claim 10, wherein the first probe corresponding to the missense mutation and the nonsense mutation in the CD79B gene, the second probe corresponding to the silent mutation in the CD79B gene, and the wild-type probe corresponding to a wild-type corresponding to these missense mutation, nonsense mutation, and silent mutation comprise the base sequences shown in Table 1.
**[Table 1]**
| Probe type | Probe base sequence | SEQ ID NO: |
|---|---|---|
| Wild-type probe | CTTACCTCGTAGGTGTGAT | 10 |
| First probe | CTTACCTCGTGGGTGTGA | 14 |
| First probe | CTTACCTCGTTGGTGTGA | 19 |
| First probe | CTTACCTCGTCGGTGTGA | 24 |
| First probe | TTACCTCGGAGGTGTGAT | 32 |
| First probe | CTCGCAGGTGTGATCTT | 38 |
| First probe | CTTACCTCGAAGGTGTGAT | 41 |
| First probe | CTTACCTCCTAGGTGTGATCT | 50 |
| First probe | TCTCCTTACCTCTTAGGTGTG | 57 |
| Second probe | TCTCCTTACCTCATAGGTGTG | 66 |
